# EUROPEAN PATENT SPECIFICATION

(11) **EP 3 387 108 B1**
(45) Date of publication and mention of the grant of the patent: **25.05.2022**
(21) Application number: 16873597.5
(22) Date of filing: 28.11.2016
(51) Int. Cl.: C12M 1/34, C12Q 1/68, C12Q 1/6816, C12Q 1/6869

(54) **METHOD OF TRANSLOCATING NUCLEIC ACIDS THROUGH NANOPORES**
VERFAHREN ZUR VERLAGERUNG VON NUKLEINSÄUREN DURCH NANOPOREN
PROCÉDÉ DE TRANSLOCATION D'ACIDES NUCLÉIQUES VIA DES NANOPORES

(30) Priority: 08.12.2015 US 201562264727 P; 10.08.2016 US 201662372928 P
(43) Date of publication of application: 17.10.2018
(73) Proprietor: Quantapore, Inc., South San Francisco, CA 94080 (US)
(72) Inventor: HONG, Tao, South San Francisco, CA 94080 (US); GUEGLER, Karl, South San Francisco, CA 94080 (US); SIMONS, Jan F., South San Francisco, CA 94080 (US)
(74) Representative: Boult Wade Tennant LLP
(86) International application number: PCT/US2016/063877
(87) International publication number: WO 2017/100027

(56) References cited:
- WO-A1-2013/185137
- WO-A1-2015/167972
- WO-A1-2017/123647

## Description

### BACKGROUND

DNA sequencing technologies developed over the last decade have revolutionized the biological sciences, e.g. van Dijk et al, Trends in Genetics, 30(9): 418-426 (2014). However, there remains a host of challenges to achieving the full potential of the technology, including reduction of per-run sequencing cost, simplification of sample preparation, reduction of run times, increasing sequence read lengths, improving data analysis, and the like. Single molecule sequencing techniques, such as nanopore-based sequencing, may address some of these challenges; however, these approaches have their own set of technical difficulties, such as, reliable nanostructure fabrication, control of DNA translocation rates, nucleotide discrimination, detection of electrical signals from large arrays of nanopore sensors, and so on, e.g. Branton et al, Nature Biotechnology, 26(10): 1146-1153 (2008). In particular, a wide variety of solutions have been proposed for controlling DNA translocation rates that range from increasing viscosity to adding molecular motors and/or DNA "ratcheting" structures to nanopores. Unfortunately, these approaches to the translocation problem present serious trade-offs with simplicity of sample preparation, ease of device fabrication, detection sensitivity, and the like. WO2013/185137 discloses modified base detection with nanopore sequencing.

In view of the above, it would be advantageous to nanopore sensor technology in general and its particular applications, such as optically based nanopore sequencing, if methods were available for conveniently and efficiently preparing nucleic acid target molecules for translocation and analysis using nanopores.

### SUMMARY OF THE INVENTION

The invention provides a method of analyzing a nucleic acid comprising: (a) extending a primer having a 5' non-complementary tail on a template in a reaction mixture to produce a double stranded product comprising a labeled extended strand and the 5' non-complementary tail as a single stranded overhang, wherein labels of said labeled extended strand are optical labels each capable of generating an optical signal indicative of a nucleotide to which it is attached; (b) providing at least one nanopore that provides fluid communication between a first chamber and a second chamber, wherein each nanopore of the at least one nanopore is capable of passing a single stranded nucleic acid but not a double stranded nucleic acid; (c) disposing the double stranded product in the first chamber; (d) capturing by the at least one nanopore the 5' non-complementary tail of the double stranded product by applying an electrical field across the nanopore; (e) translocating at a rate of less than 1000 nucleotides per second (nt/sec) the labeled extension strand of the captured double stranded product through the nanopore by the applied electrical field, wherein the translocating strand of the double stranded product is unzipped as it enters the nanopore; (f) detecting optical signals from the optical labels as said labeled extension strand passes through said nanopore; and (g) determining a nucleotide sequence of the nucleic acid from the optical signals detected in step (f).

The invention also provides a method of analyzing a nucleic acid comprising: (a) extending a primer on a template in a reaction mixture to produce a double stranded product comprising a labeled extended strand with a free 3'-hydroxyl, wherein said labels of said labeled extended strand are optical labels each capable of generating an optical signal indicative of a nucleotide to which it is attached; (b) extending further the extended strand without a template with a terminal transferase activity to produce a 3'-single stranded tail on the double stranded product; (c) providing at least one nanopore that separates and provides fluid communication between a first chamber and a second chamber, wherein each nanopore of the at least one nanopore is capable of passing a single stranded nucleic acid but not a double stranded nucleic acid; (d) disposing double stranded product with the 3'single stranded tails in the first chamber; (e) capturing a 3' single stranded tail of a double stranded product by the at least one nanopore by applying an electrical field across the nanopore; (f) translocating at a rate of less than 1000 nucleotides per second (nt/sec) the labeled extension strand of the captured double stranded product through the nanopore by the applied electrical field, wherein the translocating strand of the double stranded product is unzipped as it enters the nanopore; (g) detecting optical signals from the optical labels as said labeled extension strand passes through said nanopore; and (h) determining a nucleotide sequence of the nucleic acid from the optical signals detected in step (g).

The invention further provides a method of determining a nucleotide sequence of a polynucleotide, the method comprising the steps of: (a) providing a labeled double stranded products of target polynucleotides, wherein a labeled strand of each labeled double stranded product comprises a single stranded overhang and wherein different kinds of nucleotides of the labeled strand have different optical labels that generate distinct optical signals; (b) providing at least one nanopore that provides fluid communication between a first chamber and a second chamber, wherein each nanopore is capable of passing a single stranded nucleic acid but not a double stranded nucleic acid; (c) disposing the labeled double stranded product in the first chamber; (d) capturing by the at least one nanopore a single stranded overhang of a labeled double stranded product by applying an electrical field across the nanopore; (e) translocating the labeled stand through the nanopore so that the nucleotides of the labeled strand pass single file through an excitation zone at a rate of less than 1000 nucleotides per second (nt/sec), wherein optical labels are excited to generate optical signals and wherein the translocating labeled strand of the double stranded product is unzipped as it enters the nanopore; (f) detecting a time series of optical signals from the optical labels as the labeled strand translocates through the nanopore to produce a strand optical signature; and (g) determining a sequence of the target polynucleotide from the strand optical signature.

The present disclosure is directed to methods for preparation of polynucleotide target molecules for translocation and analysis by nanopores.

The present disclosure describes preparing double stranded DNA products comprising a labeled strand which includes a single stranded overhang which is capable of being captured by a nanopore to initiate translocation.

Described herein is a method of analyzing a nucleic acid comprising the steps of (a) extending a primer having a 5' non-complementary tail on a template in a reaction mixture to produce a double stranded product comprising an extended strand and the 5' non-complementary tail as a single stranded overhang; (b) providing a nanopore that separates and provides fluid communication between a first chamber and a second chamber, wherein the nanopore is capable of passing a single stranded nucleic acid but not a double stranded nucleic acid; (c) disposing the double stranded product in the first chamber; (d) capturing the 5' non-complementary tail of the double stranded product by the nanopore by applying an electrical field across the nanopore; and (d) translocating at a detectable rate the labeled extension strand of the captured double stranded product through the nanopore by the applied electrical field, wherein the translocating strand of the double stranded product is unzipped, or rendered single stranded, as it enters the nanopore. In some embodiments, methods of the invention further include a step of isolating the double stranded product from the extension reaction mixture prior to disposing the double stranded product in the first chamber.

Described herein is a method of analyzing a nucleic acid comprising the steps of: (a) extending a primer on a template in a reaction mixture to produce a double stranded product comprising a labeled extended strand with a free 3'-hydroxyl; (b) extending further the extended strand without a template with a terminal transferase activity to produce a 3'-single stranded tail on the double stranded product; (c) providing at least one nanopore that separates and provides fluid communication between a first chamber and a second chamber, wherein each nanopore of the at least one nanopore is capable of passing a single stranded nucleic acid but not a double stranded nucleic acid; (d) disposing double stranded product with the 3'single stranded tails in the first chamber; (e) capturing a 3' single stranded tail of a double stranded product by the at least one nanopore by applying an electrical field across the nanopore; (f) translocating at a rate of less than 1000 nucleotides per second (nt/sec) the labeled extension strand of the captured double stranded product through the nanopore by the applied electrical field, wherein the translocating strand of the double stranded product is unzipped as it enters the nanopore.

The present invention solves several problems related to nanopore-based analysis of nucleic acids including, but not limited to, reducing translocation speed to improve analysis without complicating sample preparation steps, eliminating the need for removing template strands or for providing nucleic acid-denaturing conditions (e.g. 4M urea) for analysis, providing the same 5' to 3' translocation orientation of target nucleic acids for simplified data analysis, and the like. These and other advantages of the present invention are exemplified in a number of implementations and applications, some of which are summarized below and throughout the specification.

### BRIEF DESCRIPTION OF THE DRAWINGS

Figs. 1A-1F illustrate steps of different embodiments of the present invention.
Figs. 2A-2D illustrate a hybrid nanopore configuration for optically based nucleic acid analysis.
Figs. 3A-3C illustrate two-color nanopore sequencing methods that employ the present invention.
Fig. 4 illustrates a method for optically based nanopore analysis of nucleic acids which employs the present invention.
Fig. 5 shows trans-nanopore current data for one embodiment of the invention.

### DETAILED DESCRIPTION OF THE INVENTION

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention. For example, particular nanopore types and numbers, particular labels, FRET pairs, detection schemes, fabrication approaches of the invention are shown for purposes of illustration. It should be appreciated, however, that the disclosure is not intended to be limiting in this respect, as other types of nanopores, arrays of nanopores, and other fabrication technologies may be utilized to implement various aspects of the systems discussed herein. Guidance for aspects of the invention is found in many available references and treatises well known to those with ordinary skill in the art, including, for example, Cao, Nanostructures & Nanomaterials (Imperial College Press, 2004); Levinson, Principles of Lithography, Second Edition (SPIE Press, 2005); Doering and Nishi, Editors, Handbook of Semiconductor Manufacturing Technology, Second Edition (CRC Press, 2007); Sawyer et al, Electrochemistry for Chemists, 2nd edition (Wiley Interscience, 1995); Bard and Faulkner, Electrochemical Methods: Fundamentals and Applications, 2nd edition (Wiley, 2000); Lakowicz, Principles of Fluorescence Spectroscopy, 3rd edition (Springer, 2006); Hermanson, Bioconjugate Techniques, Second Edition (Academic Press, 2008); and the like.

The present invention is directed to methods of nanopore-based analysis of nucleic acids that use the duplex bond energy of prepared nucleic acid analytes to control nanopore translocation speed. In some embodiments, features of the invention include (i) using a nanopore having a bore, or aperture, that (under conditions of operation) can discriminate between single stranded nucleic acids and double stranded nucleic acids by allowing passage of single stranded nucleic acids but not allowing passage of double stranded nucleic acids, and (ii) preparing nucleic acid analytes in the form of a double stranded product that has a single stranded tail, or overhang, that permits capture by a nanopore under conditions of operation. In some embodiments, the single stranded tail may be a charged polymer, such as a single stranded polynucleotide, and conditions of operation include exposing the double stranded products to the nanopore in an electrolyte solution and establishing an electrical field, or voltage gradient, across the nanopore so that the charged polymer tail is driven by the electric field through the nanopore after capture. In embodiments where the single stranded tail is a polynucleotide, the single stranded tail of the double stranded product that is captured by a nanopore may be a 3'-overhang of the double stranded product or a 5'-overhang of the double stranded product.

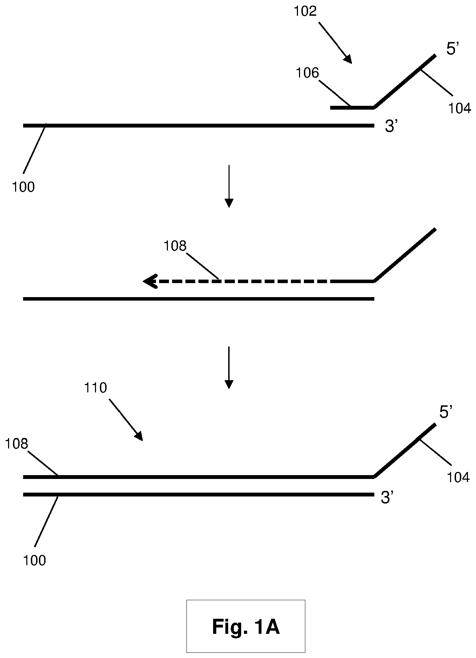

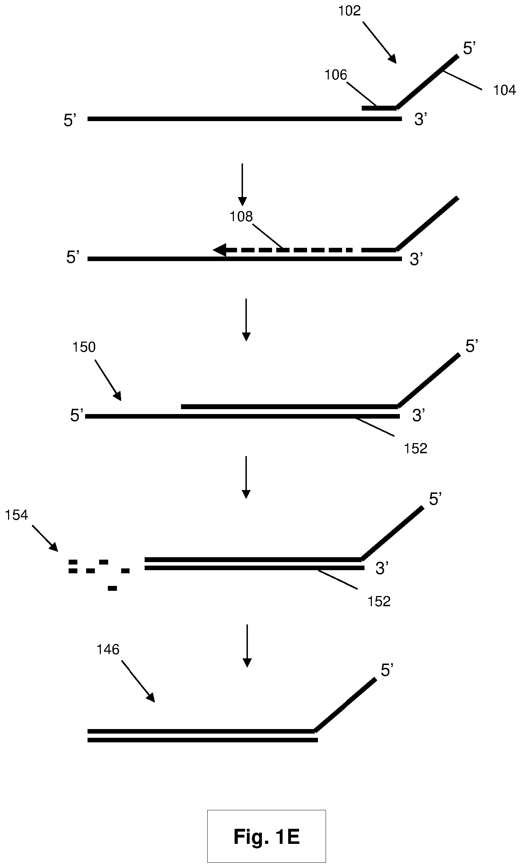

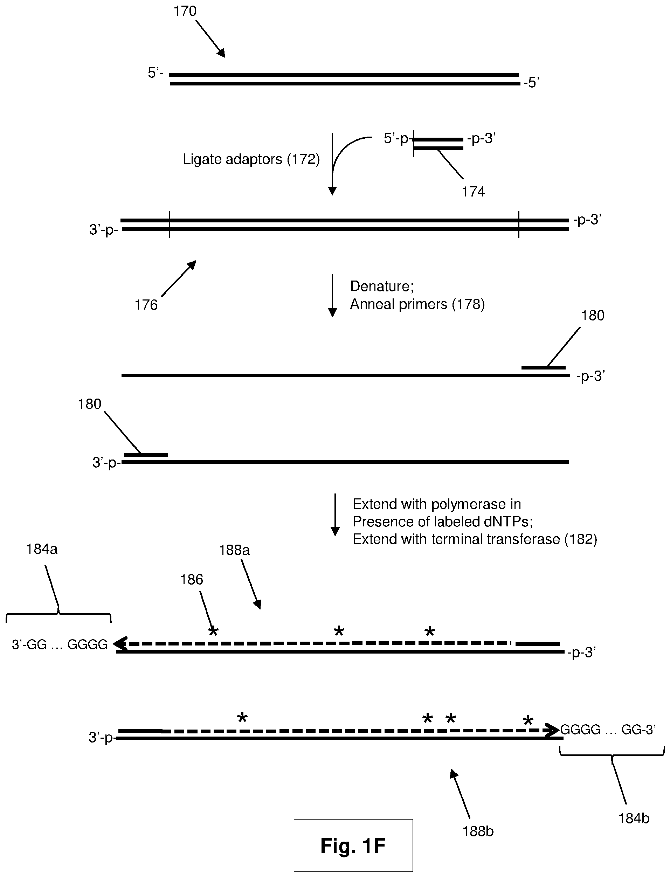

Some embodiments of the invention are illustrated by Fig. 1A. Template nucleic acid (100) is combined with primer (102) under primer-annealing conditions that permit template-specific portion (106) of primer (102) to anneal to template (100). Primer (102) comprises template-specific portion (106) that may be extended by a nucleic acid polymerase, such as a DNA polymerase, and tail (104) that is a charged polymer under conditions of operation. Template-specific portion (106) is at the 3' end of primer (102) and is extendable by a nucleic acid polymerase. Tail (104) is at the 5' end of primer (102). In some embodiments, the binding site of template-specific portion (106) may include the 3' end of template (100) so that there is no 3' overhang, as shown in Fig. 1A. In other embodiments, the binding site of template-specific portion (106) may be inset from the 3' end of template (100) so that there is a small 3' overhang (that is, "small" relative to the size of the 5' overhang of the other strand) (not shown). In some embodiments, such 3' overhang of template strand (106) may be in the range of from 1 to 5 nucleotides, or from 1 to 3 nucleotides. While not intending to be bound by theory, it is believed that the small 3' overhang of the template strand may facilitate the beginning of the unzipping event after a 5' tail of a double stranded product is captured by a nanopore. The length of template-specific portion (106) is conventional and may be selected in the range specified in the "primer" definition section. In other embodiments, the binding site of template-specific portion (106) may be at an interior location of template (100), as described below. In some embodiments, the sequence of template-specific portion (106) is predetermined, for example, because templates (100) had been previously amplified using adaptor sequences containing primer binding sites for the template-specific portion (106). In other embodiments, there may be a plurality of template-specific portions, each predetermined and each specific for a separate template sequence (106). In such latter embodiments, the plurality of primers with different template-specific portions may vary widely; for example, such plurality may be in the range of from 2 to 1000, or in the range of from 2 to 500, or in the range of from 10 to 1000.

In some embodiments, the sequence of template-specific portion (106) may be a random sequence, e.g. so that the sequence of primer (102) has a formula: 3'-NNN ... NN-nnn ... nn-5', wherein each N is A, C, G or T and each n is a tail monomer, such as T. In some embodiments, the sequence of primer (102) has a formula: 3'-NNN ... NN-αnnn ... nn-5', wherein each N is A, C, G or T, α is one or more extension-blocking monomers, such abasic nucleotides, and n is a tail monomer, such as T. In some embodiments, α comprises from 1 to 6 extension-blocking monomers.

Tail (104) may comprise a single stranded nucleotide chain that is preferably not complementary to other template sequences or self-complementary. Alternatively, tail (102) may comprise a charged polymer that is, in whole or in part, a chain of non-nucleosidic monomers, such as abasic ribose monomers connected by phosphodiester linkages. In some embodiments, the net charge of tail (104) is negative. In some embodiments, the net charge of tail (104) is sufficiently high to overcome resistance by the nanopore to single strand translocation and to unzip the double stranded portion of double stranded product (110). Whenever tail (104) comprises nucleotides or monomers linked by phosphodiester bonds, the length of tail (104) may vary widely and is constrained, on the one hand (at the shorter lengths), by the amount of charge necessary for translocation and unzipping, and on the other hand (at the longer lengths) by convenience and cost of synthesis. In some embodiments, tail (104) has a length of at least 10 monomers; in other embodiments, tail (104) has a length in the range of from 10 to 120 monomers. After primers (102) anneal to templates (100), primer extension conditions are established (if not already present during annealing) so that extended strands (108) complementary to templates (100) are synthesized and double stranded product (110) is made. In some embodiments, primer extension conditions comprise conventional polymerase reaction conditions; that is, the presence of a nucleic acid polymerase and appropriate nucleoside triphosphate monomers at proper pH, salt concentration, and the like, for template-based synthesis of extended strand (108) starting from template-specific portion (106) of primer (102). Such synthesis results in double stranded product (110) that comprises a duplex portion comprising original template strand (100) duplexed with extended strand (108) and template-specific portion (106) and single stranded tail (104).

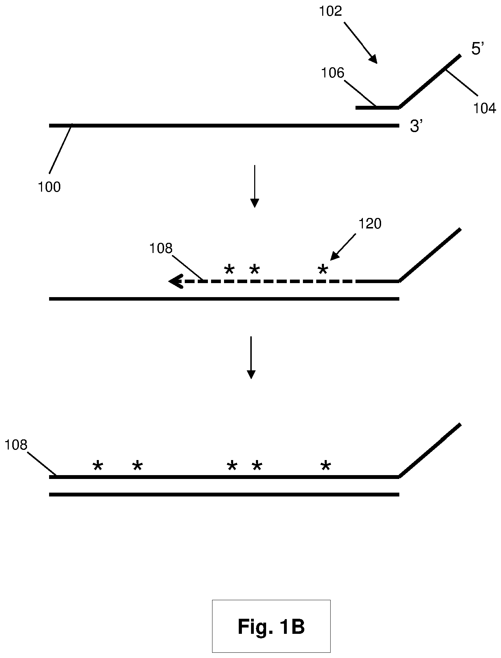

As indicated in Fig. 1B, extended strand (108) may be labeled by the incorporation of modified nucleotide precursor that provides a label (120) either directly, e.g. by a fluorescent moiety already attached to the precursor, or indirectly, e.g. by a reactive group that is used later (e.g. after a primer extension reaction) to attached a label having a complementary group. In some embodiments, modified nucleotide precursors are employed so that every incorporated nucleotide of extended stand (108) is directly or indirectly labeled. In some embodiments, at least two direct or indirect labels are used. In still other embodiments, at least four labeling reactions are performed so that on average four sets of extended strands (108) are produced, wherein in a first set the labels represent the presence of an "A" or a "not-A", in a second set the labels represent the presence of a "C" or a "not-C", in a third set the labels represent the presence of a "G" or a "not-G", and in a fourth set the labels represent the presence of a "T" or a "not-T". In some embodiments, 3 of the 4 different kinds of nucleotide may be labeled; and in some embodiments, 2 of the 4 different kinds of nucleotide may be labeled, such as, all, or substantially all, of the pyrimidines, T and C (and wherein substantially all the purines are unlabeled).

Template (100) may be used in methods of the invention after being obtained directly from a sample; or, in some embodiments, steps of the method may be performed in situ, after which double stranded product is extracted for analysis. In other embodiments, template (100) may be a product of additional procedures performed before implementing steps of the invention, such as extracting nucleic acids from a sample, amplification of nucleic acids from the sample, or the like. In some embodiments, templates (100) are produced by multiplex PCR of a plurality of selected target nucleic acids; in other embodiments, templates (100) are produced by a whole genome amplification.

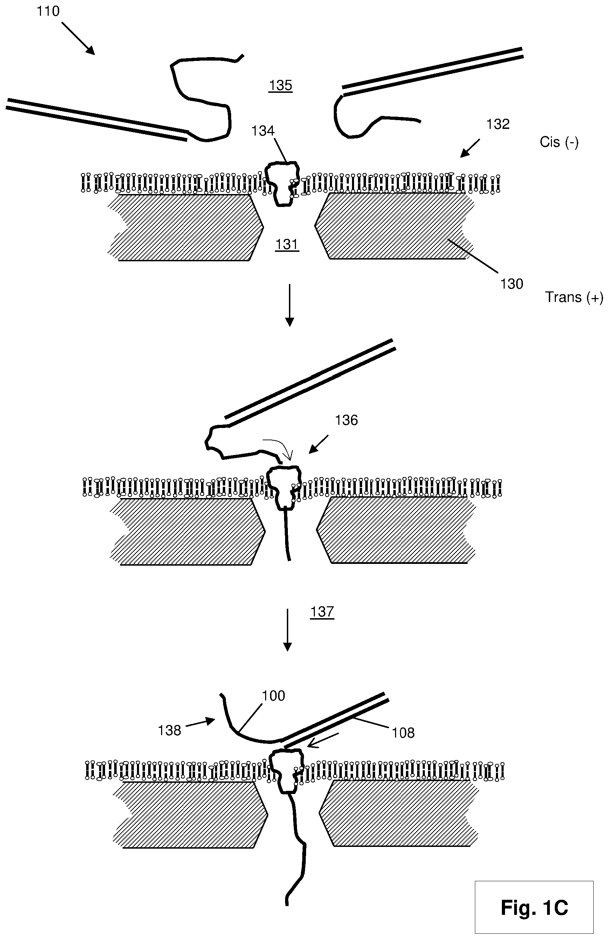

After the extension reaction illustrated in Figs. 1A or 1B is completed, double stranded product (110) is exposed in a translocation reaction mixture to one or more nanopores in a nanopore sequencing device. In some embodiments, such translocation reaction mixture is a conventional electrolyte used for translocating nucleic acid strands through nanopores, wherein the ionic strength (mono- and/or divalent cation concentrations) in the first chamber is selected (along with electrical field strength across nanopores) so that target polynucleotides remain double stranded upon deposition therein and are unzipped during translocation under the selected electrical field strength. Briefly, and without intending to be limiting, such a device comprises a first chamber and a second chamber separated by an impermeable membrane containing one or more nanopores, such that fluid communication between the first chamber and second chamber is solely through the one or more nanopores. Such a device typically also includes a set of electrodes for establishing an electric field across the membrane and nanopore(s). The electric field used to move, or translocate, polynucleotides (that are negatively charged in a selected electrolyte reaction mixture) from the first chamber to the second chamber through the nanopores. In some embodiments, the membrane includes a plurality of nanopores arranged as an array. For example, in Fig. 1C, protein nanopore (134) is positioned at aperture (131) in lipid bilayer (132) which is dispose on a surface of membrane (130). Double stranded products (110) are exposed to nanopore (134) by placing them in an electrolyte in first chamber (135), which is configured as the "cis" side of membrane (130) by placement of a negative electrode (not shown) in the chamber. Upon application of an electric field, a negatively charged tail (104) captured by nanopore (134) will translocate (136) through nanopore (134) to second chamber (137), which is configured as the "trans" side of membrane (130) by placement of a positive electrode in the chamber. The translocation continues and the force exerted by the translocating tail (104) initiates the unzipping (138) of template strand (100) from extended strand (108). The speed of translocation depends in part on the ionic strength of the electrolytes in the first and second chambers and the applied voltage across the nanopores. In optically based detection, a translocation speed may be selected by preliminary calibration measurements, for example, using predetermined standards of double stranded products that generate signals at different expected rates per nanopore for different voltages. Thus, for DNA sequencing applications, a translocation speed may be selected based on the signal rates from such calibration measurements. Consequently, from such measurements a voltage and/or ionic strength may be selected that permits, or maximizes, reliable nucleotide identifications, for example, over an array of nanopores. In some embodiments, such calibrations may be made using double stranded products from the sample of templates being analyzed (instead of, or in addition to, predetermined standard sequences). In some embodiments, such calibrations may be carried out in real time during a sequencing run and the applied voltage may be modified in real time based on such measurements, for example, to maximize the acquisition of nucleotide-specific signals.

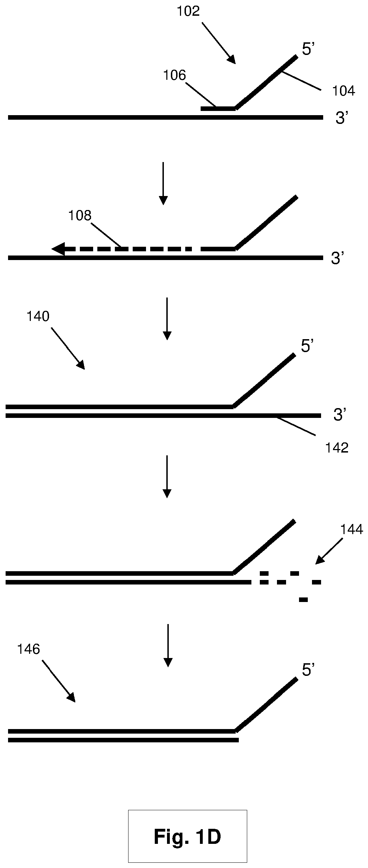

As noted above, in some embodiments, binding sites of template-specific portions (106) of primer (102) may be in the interior of templates (100), as illustrated in Fig. 1D. Thus, after extension of annealed primer (102) to form extended strand (108), initial double stranded product (140) is formed that has single stranded 5' tail (104) and single stranded 3' tail (142). In some embodiments, such initial double stranded product (140) may be used in accordance with the invention; that is, it may be disposed in the first chamber for capture by nanopores either via its 5' tail (104) or its 3' tail (142), after which translocation and unzipping takes place. In other embodiments, 3' tail (142) may be digested (144) with a single stranded 3' exonuclease, such as a T4 DNA polymerase, to produce double stranded product (146) whose sole overhang is 5' tail (104).

In some embodiments, as illustrated in Fig. IE, extended strand (108) may not extend to the end of template (152) leaving 5' single stranded overhang (150) of template (152). In such cases, the undesired 5' end (150) of template may be treated with a 5' single stranded exonuclease, such as mung bean nuclease, to produce double stranded product (146). The desired 5' tail of extended strand (108) may be protected from digestion by incorporating nuclease-resistance nucleotide analogs, such as phosphorothioates.

In some embodiments, a double stranded product may have a 3' single stranded overhang, as illustrated in Fig. IF. Double stranded products with 3' single stranded overhangs allow labeled strands to be captured by their 3' ends and to translocate nanopores in a 3'-first orientation, which progresses at a different speed than translocation in a 5'-first orientation depending on the nanopore being used. For example, in some embodiments employing an α-hemolysin nanopore, a 3'-first translocation speed may be less than or equal to 50 percent that of 5'-first translocation speed. Briefly, in this embodiment, 3' single stranded overhangs are created by template-free extension using a terminal transferase (TdT) activity. In one implementation, adaptors (174) are ligated (172) to blunt-ended target polynucleotide (170) to produce double stranded product (176). Adaptor (174) comprises one end that is capable of participating in a blunt-end ligation reaction and another end that can neither be ligated nor extended by terminal transferase. In some embodiments, these capabilities are implemented by providing a 5' phosphate group (indicated by "p" in Fig. IF) at one end and a 3' phosphate group (indicated by "p" in Fig. IF) at the other end of the same strand of adaptor (174). Strands with both 3' and 5' phosphates are readily synthesized using commercially available phosphoramidite chemistries. Optionally, a step of size separation may be included to separate product (176) from self-ligated adaptor-adaptor side products. The large double stranded product of the ligation reaction is denatured and primers (180) are annealed (178) to primer binding sites located in adaptor strands with 3'-blocked ends. After such annealing, primers (180) are extended in the presence of selected labeled and/or unlabeled dNTP analogs (182) to form a labeled (e.g. 186) double stranded product. Either in a separate reaction or in the same reaction, free (unblocked) 3'-ends of the labeled double stranded products are further extended by a terminal transferase. In some embodiments in which two-color sequencing is used (see below), a terminal transferase extension may take place in a separate reaction so that nucleotides of the 3' single stranded overhang are unlabeled. Terminal transferase extension produces labeled double stranded products (188a) and (188b) that have single stranded overhangs (184a and 184b, respectively). The lengths of single stranded extensions (184a and 184b) may vary widely. In some embodiment, lengths of single stranded extension (184a and/or 184b) are each at least 10 nucleotides; in other embodiments, lengths of single stranded extension (184a and/or 184b) are each at least 20 nucleotides; in other embodiments, lengths of single stranded extension (184a and/or 184b) are each in a range of from 10 to 120 nucleotides.

As mentioned above, any of the above embodiments may include a further step of isolating or purifying or separating (from the extension reaction mixture) the double stranded product prior to exposing them to a nanopore or an array of nanopores. Such isolation, or separation, may be carried out using conventional double stranded DNA isolation techniques or kits, such as employed for isolating PCR products, e.g. QIAquick PCR purification kit (Qiagen, Inc., Valencia, CA).

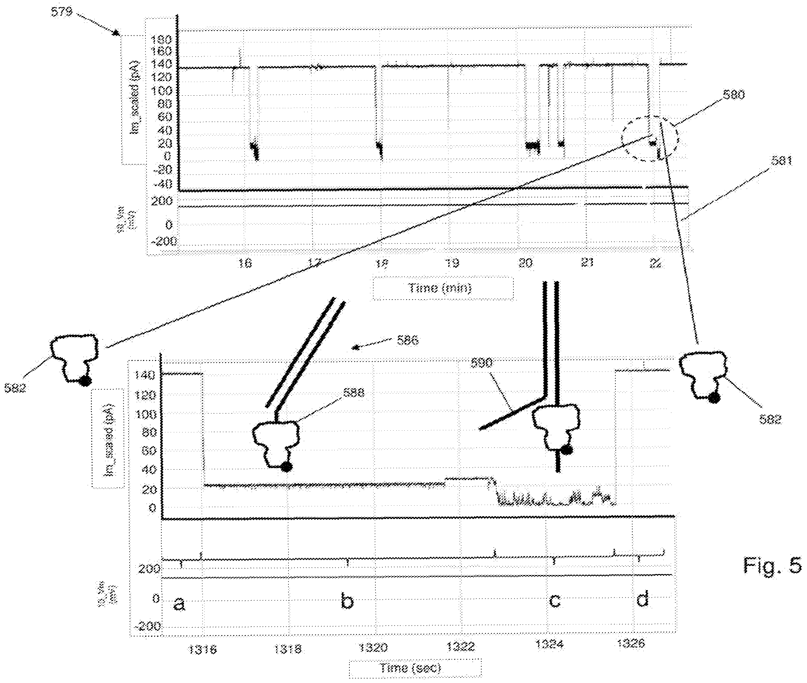

As mentioned above, translocation speeds depend in part on the voltage difference (or electrical field strength) across a nanopore and conditions in the reaction mixture of the first chamber where double stranded product is exposed to the nanopore. Double stranded product capture rates depend on concentration of such products. In some embodiments, conventional reaction mixture conditions for nanopore sequencing may be employed with the invention, for example, 1M KCl (or equivalent salt, such as NaCl, LiCl₂, or the like) and a pH buffering system (which, for example, ensures that proteins being used, e.g. protein nanopores, nucleases, or the like, are not denatured). In some embodiments, a pH buffering system may be used to keep the pH substantially constant at a value in the range of 6.8 to 8.8. In some embodiments, a voltage difference across the nanopores may be in the range of from 70 to 200 mV. In other embodiments, a voltage difference across the nanopores may be in the range of from 80 to 150 mV. An appropriate voltage for operation may be selected using conventional measurement techniques, as illustrated in Fig. 5. Current (or voltage) across a nanopore may readily be measured using commercially available instruments. Chart (579) shows current values versus time of the current across a nanopore when double stranded products (586) are captured and unzipped and extended stands are translocated through the nanopore. A test double stranded product was used to generate the data shown, which consisted of a double stranded portion of approximately 510 basepairs of roughly equal proportions of A's, C's, G's and T's, and a 5' poly-T tail of 40 nucleotides. (Template strand of double stranded product was the same that disclosed in International patent publication WO2014/190322, which is incorporated herein by reference). Blow-up (581) of current drop (580) shows what are believed to be the different phases (a, b, c and d) of capture and translocation along with their associated current levels. Current level or phase (a) corresponds to unoccupied nanopore (582). Current level or phase (b) corresponds to nanopore (583) occupied by double strand product (586) captured by strand (588) but that has not yet undergone unzipping. Current level or phase (c) corresponds to double stranded product (586) undergoing unzipping with the release of strand (590). Current level or phase (d) corresponds to nanopore (582) returning to an unoccupied state. Using measurements such as these with a predetermined standard for double stranded products, a nucleotide per second translocation speed may be determined from the time interval corresponding to phase (c). A voltage difference may be selected so that translocation speed is within a desired range. In some embodiments, a range of translocation speeds comprises those speeds less than 1000 nucleotides per second. In other embodiments, a range of translocation speeds is from 10 to 800 nucleotides per second; in other embodiments, a range of translocation speeds is from 10 to 600 nucleotides per second; in other embodiments, a range of translocation speeds is from 200 to 800 nucleotides per second; in other embodiments, a range of translocation speeds is from 200 to 500 nucleotides per second.

### Nanopores and Nanopore Arrays

Nanopores used with the invention may be solid-state nanopores, protein nanopores, or hybrid nanopores comprising protein nanopores or organic nanotubes such as carbon or graphene nanotubes, configured in a solid-state membrane, or like framework. Important features of nanopores include (i) constraining polymer analytes, such as polynucleotides, so that their monomers pass through a detection zone in sequence (that is, so that nucleotides pass a detection zone one at a time, or in single file) and (ii) passing single stranded nucleic acids while not passing double stranded nucleic acids, or equivalently bulky molecules.

In some embodiments, nanopores used in connection with the methods of the invention are provided in the form of arrays, such as an array of clusters of nanopores, which may be disposed regularly on a planar surface. In some embodiments, clusters are each in a separate resolution limited area so that optical signals from nanopores of different clusters are distinguishable by the optical detection system employed, but optical signals from nanopores within the same cluster cannot necessarily be assigned to a specific nanopore within such cluster by the optical detection system employed.

Solid state nanopores may be fabricated in a variety of materials including but not limited to, silicon nitride (Si₃N₄), silicon dioxide (SiO₂), and the like. The fabrication and operation of nanopores for analytical applications, such as DNA sequencing, are disclosed in the following exemplary references that are incorporated by reference: Ling, U.S. patent 7,678,562; Hu et al, U.S. patent 7,397,232; Golovchenko et al, U.S. patent 6,464,842; Chu et al, U.S. patent 5,798,042; Sauer et al, U.S. patent 7,001,792; Su et al, U.S. patent 7,744,816; Church et al, U.S. patent 5,795,782; Bayley et al, U.S. patent 6,426,231; Akeson et al, U.S. patent 7,189,503; Bayley et al, U.S. patent 6,916,665; Akeson et al, U.S. patent 6,267,872; Meller et al, U.S. patent publication 2009/0029477; Howorka et al, International patent publication WO2009/007743; Brown et al, International patent publication WO2011/067559; Meller et al, International patent publication WO2009/020682; Polonsky et al, International patent publication WO2008/092760; Van der Zaag et al, International patent publication WO2010/007537; Yan et al, Nano Letters, 5(6): 1129-1134 (2005); Iqbal et al, Nature Nanotechnology, 2: 243-248 (2007); Wanunu et al, Nano Letters, 7(6): 1580-1585 (2007); Dekker, Nature Nanotechnology, 2: 209-215 (2007); Storm et al, Nature Materials, 2: 537-540 (2003); Wu et al, Electrophoresis, 29(13): 2754-2759 (2008); Nakane et al, Electrophoresis, 23: 2592-2601 (2002); Zhe et al, J. Micromech. Microeng., 17: 304-313 (2007); Henriquez et al, The Analyst, 129: 478-482 (2004); Jagtiani et al, J. Micromech. Microeng., 16: 1530-1539 (2006); Nakane et al, J. Phys. Condens. Matter, 15 R1365-R1393 (2003); DeBlois et al, Rev. Sci. Instruments, 41(7): 909-916 (1970); Clarke et al, Nature Nanotechnology, 4(4): 265-270 (2009); Bayley et al, U.S. patent publication 2003/0215881; and the like.

Briefly, in some embodiments, a 1-100 nm channel or aperture is formed through a substrate, usually a planar substrate, such as a membrane, through which an analyte, such as single stranded DNA, is induced to translocate. In other embodiments, a 2-50 nm channel or aperture is formed through a substrate; and in still other embodiments, a 2-30 nm, or a 2-20 nm, or a 3-30 nm, or a 3-20 nm, or a 3-10 nm channel or aperture if formed through a substrate. The solid-state approach of generating nanopores offers robustness and durability as well as the ability to tune the size and shape of the nanopore, the ability to fabricate high-density arrays of nanopores on a wafer scale, superior mechanical, chemical and thermal characteristics compared with lipid-based systems, and the possibility of integrating with electronic or optical readout techniques. Biological nanopores on the other hand provide reproducible narrow bores, or lumens, especially in the 1-10 nanometer range, as well as techniques for tailoring the physical and/or chemical properties of the nanopore and for directly or indirectly attaching groups or elements, such as fluorescent labels, which may be FRET donors or acceptors, by conventional protein engineering methods. Protein nanopores typically rely on delicate lipid bilayers for mechanical support, and the fabrication of solid-state nanopores with precise dimensions remains challenging. In some embodiments, solid-state nanopores may be combined with a biological nanopore to form a so-called "hybrid" nanopore that overcomes some of these shortcomings, thereby providing the precision of a biological pore protein with the stability of a solid state nanopore. For optical read out techniques a hybrid nanopore provides a precise location of the nanopore which simplifies the data acquisition greatly.

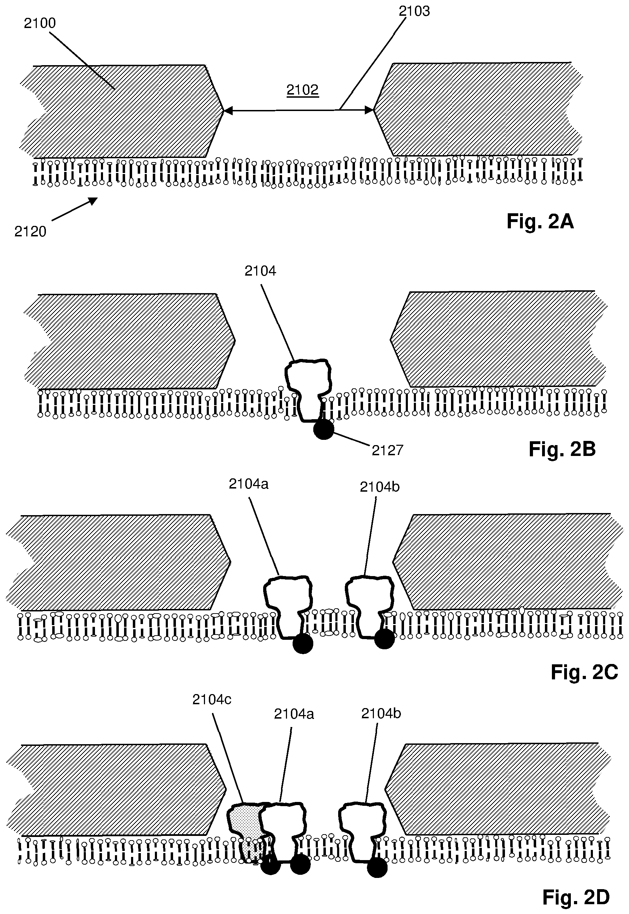

In some embodiments, clusters may also be formed by disposing protein nanopores in lipid bilayers supported by solid phase membrane containing an array of apertures. For example, such an array may comprise apertures fabricated (e.g. drilled, etched, or the like) in solid phase support (2102 in Fig. 2A). The geometry of such apertures may vary depending on the fabrication techniques employed. In some embodiments, each such aperture is associated with, or encompassed by, a separate resolution limited area; however, in other embodiments, multiple apertures may be within the same resolution limited area. The cross-sectional area of the apertures may vary widely and may or may not be the same as between different clusters, although such areas are usually substantially the same as a result of conventional fabrication approaches. In some embodiments, apertures have a minimal linear dimension (2103) (e.g. diameter in the case of circular apertures) in the range of from 10 to 200 nm, or have areas in the range of from about 100 to 3×10⁴ nm². Across the apertures is disposed a lipid bilayer, illustrated in cross-section in Figs. 2A-2D. In some embodiments, such lipid bilayer (2120) is disposed over one surface of solid phase membrane (2100). In some embodiments, protein nanopores (2104 in Figs. 2A-2D) are inserted into portions of lipid bilayer (2120) spanning the apertures, where in some embodiments, such as those depicted, protein nanopores may be directly labeled (2127), e.g. with a FRET donor. In some embodiments, such protein nanopores are inserted from solution in a chamber on one side of solid phase membrane (2100), which results in a random placement of protein nanopores into the aperture, such that different apertures may receive different numbers of protein nanopores, as illustrated in Figs. 2A-2D, where apertures are shown with no, one, two, or three protein nanopores. The distribution of protein nanopores per aperture may be varied, for example, by controlling the concentration of protein nanopores during inserting step. In such embodiments, clusters of nanopores may comprise a random number of nanopores. In some embodiments, in which protein nanopores insert randomly into apertures, clusters containing one or more apertures on average have a number of protein nanopores that is greater than zero; in other embodiments, such clusters have a number of protein nanopores that is greater than 0.25; in other embodiments, such clusters have a number of protein nanopores that is greater than 0.5; in other embodiments, such clusters have a number of protein nanopores that is greater than 0.75; in other embodiments, such clusters have a number of protein nanopores that is greater than 1.0.

In some embodiments, devices for use in the methods of the invention comprise a solid phase membrane, such as a SiN membrane, having an array of apertures therethrough providing communication between a first chamber and a second chamber (also sometimes referred to as a "cis chamber" and a "trans chamber") and supporting a lipid bilayer on a surface facing the second, or trans, chamber. In some embodiments, diameters of the aperture in such a solid phase membrane may be in the range of 10 to 200 nm, or in the range of 20 to 100 nm. In some embodiments, such solid phase membranes further include protein nanopores inserted into the lipid bilayer in regions where such bilayer spans the apertures on the surface facing the trans chamber. In some embodiments, such protein nanopores are inserted from the cis side of the solid phase membrane using techniques described herein. In some embodiments, such protein nanopores have a structure identical to, or similar to, α-hemolysin in that it comprises a barrel, or bore, along an axis and at one end has a "cap" structure and at the other end has a "stem" structure (using the terminology from Song et al, Science, 274: 1859-1866 (1996)). In some embodiments using such protein nanopores, insertion into the lipid bilayer results in the protein nanopore being oriented so that its cap structure is exposed to the cis chamber and its stem structure is exposed to the trans chamber.

In some embodiments, devices for use in methods of the invention comprise droplet interface bilayers, either as single droplets or as arrays droplets, for example, as disclosed in Bayley et al, U.S. patent publication 2014/0356289; Huang et al, Nature Nanotechnology, 10.1038/nnano.2015.189. [Epub ahead of print]; or like reference, which are hereby incorporated by reference. Briefly, protein nanopores (1.2 nM) are placed in a 200-350 nl droplet (for example, 1.32 M KCl, 8.8 mM HEPES, 0.4 mM EDTA, pH 7.0 (αHL) or 8.0 (MspA), and incubated in, for example, 3 mM 1,2-diphytanoyl-sn-glycero-3-phosphocholine (DPhPC) in hexadecane to form a lipid monolayer coating. A droplet may then be transferred by pipetting onto a coverslip in a measurement chamber, for example, that permits application of voltages to move analytes and optical detection, for example, by TIRF. The coverslip may be spin coated (3,000 r.p.m., 30 s) with a thin layer (~200 nm) of agarose (0.66 M CaCl2, 8.8 mM HEPES, pH 7.0 (αHL)/8.0 (MspA)) and subsequently incubated with 3 mM DPhPC in hexadecane. On contact with the monolayer on the agarose, a lipid coated droplet spontaneously forms a droplet interface bilayer. A ground electrode (Ag/AgCl) may be inserted into the droplet, with a corresponding active electrode (Ag/AgCl) in the substrate agarose. Voltage protocols may be applied with a patch clamp amplifier (for example, Axopatch 200B, Molecular Devices). Nanopores present in the droplet spontaneously insert into the droplet interface bilayer, and the ion flux may be detected both electrically and/or optically (for example, by way of an ion-sensitive dye, such as Fluo-8, or the like).

In some embodiments, the present invention may employ hybrid nanopores in clusters, particularly for optical-based nanopore sequencing of polynucleotides. Such nanopores comprise a solid-state orifice, or aperture, into which a protein biosensor, such as a protein nanopore, is stably inserted. A charged polymer may be attached to a protein nanopore (e.g. alpha hemolysin) by conventional protein engineering techniques after which an applied electric field may be used to guide a protein nanopore into an aperture in a solid-state membrane. In some embodiments, the aperture in the solid-state substrate is selected to be slightly smaller than the protein, thereby preventing it from translocating through the aperture. Instead, the protein will be embedded into the solid-state orifice.

In some embodiments, a donor fluorophore is attached to the protein nanopore. This complex is then inserted into a solid-state aperture or nanohole (for example, 3-10 nm in diameter) by applying an electric field across the solid state nanohole, or aperture, until the protein nanopore is transported into the solid-state nanohole to form a hybrid nanopore. The formation of the hybrid nanopore can be verified by (a) the inserted protein nanopore causing a drop in current based on a partial blockage of the solid-state nanohole and by (b) the optical detection of the donor fluorophore.

Solid state, or synthetic, nanopores may be prepared in a variety of ways, as exemplified in the references cited above. In some embodiments a helium ion microscope may be used to drill the synthetic nanopores in a variety of materials, e.g. as disclosed by Yang et al, Nanotechnology, 22: 285310 (2011). A chip that supports one or more regions of a thin-film material, e.g. silicon nitride, that has been processed to be a free-standing membrane is introduced to the helium ion microscope (HIM) chamber. HIM motor controls are used to bring a free-standing membrane into the path of the ion beam while the microscope is set for low magnification. Beam parameters including focus and stigmation are adjusted at a region adjacent to the free-standing membrane, but on the solid substrate. Once the parameters have been properly fixed, the chip position is moved such that the free-standing membrane region is centered on the ion beam scan region and the beam is blanked. The HIM field of view is set to a dimension (in µm) that is sufficient to contain the entire anticipated nanopore pattern and sufficient to be useful in future optical readout (i.e. dependent on optical magnification, camera resolution, etc.). The ion beam is then rastered once through the entire field of view at a pixel dwell time that results in a total ion dose sufficient to remove all or most of the membrane autofluorescence. The field of view is then set to the proper value (smaller than that used above) to perform lithographically-defined milling of either a single nanopore or an array of nanopores. The pixel dwell time of the pattern is set to result in nanopores of one or more predetermined diameters, determined through the use of a calibration sample prior to sample processing. This entire process is repeated for each desired region on a single chip and/or for each chip introduced into the HIM chamber.

In some embodiments, a nanopore may have one or more labels attached for use in optically-based nanopore sequencing methods. The label may be a member of a Forster Resonance Energy Transfer (FRET) pair. Such labels may comprise organic fluorophores, chemiluminescent labels, quantum dots, metallic nanoparticles and/or fluorescent proteins. Target nucleic acids may have one distinct label per nucleotide. The labels attached to the nucleotides may be selected from the group consisting of organic fluorophores. The label attachment site in the pore protein can be generated by conventional protein engineering methods, e.g. a mutant protein can be constructed that will allow the specific binding of the label. As an example, a cysteine residue may be inserted at the desired position of the protein which inserts a thiol (SH) group that can be used to attach a label. The cysteine can either replace a natural occurring amino acid or can be incorporated as an addition amino acid. A maleimide-activated label is then covalently attached to the thiol residue of the protein nanopore. In a preferred embodiment the attachment of the label to the protein nanopore or the label on the nucleic acid is reversible. By implementing a cleavable crosslinker, an easily breakable chemical bond (e.g. an S-S bond or a pH labile bond) is introduced and the label may be removed when the corresponding conditions are met.

In some embodiments, nanopore arrays comprise one or more light-blocking layers, that is, one or more opaque layers. Typically nanopore arrays are fabricated in thin sheets of material, such as, silicon, silicon nitride, silicon oxide, aluminum oxide, or the like, which readily transmit light, particularly at the thicknesses used, e.g. less than 50-100 nm. For electrical detection of analytes this is not a problem. However, in optically-based detection of labeled molecules translocating nanopores, light transmitted through an array invariably excites materials outside of intended reaction sites, thus generates optical noise, for example, from nonspecific background fluorescence, fluorescence from labels of molecules that have not yet entered a nanopore, or the like. In some embodiments, this problem may be addressed by providing nanopore arrays with one or more light-blocking layers that reflect and/or absorb light from an excitation beam, thereby reducing background noise for optical signals generated at intended reaction sites associated with nanopores of an array (for example, at a nanopore exit to, or nanopore orifice opening to, a trans chamber). In some embodiments, this permits optical labels in intended reaction sites to be excited by direct illumination. In some embodiments, an opaque layer may be a metal layer. Such metal layer may comprise Sn, Al, V, Ti, Ni, Mo, Ta, W, Au, Ag or Cu. In some embodiments such metal layer may comprise Al, Au, Ag or Cu. In still other embodiments, such metal layer may comprise aluminum or gold, or may comprise solely aluminum. The thickness of an opaque layer may vary widely and depends on the physical and chemical properties of material composing the layer. In some embodiments, the thickness of an opaque layer may be at least 5 nm, or at least 10 nm, or at least 40 nm. In other embodiments, the thickness of an opaque layer may be in the range of from 5-100 nm; in other embodiments, the thickness of an opaque layer may be in the range of from 10-80 nm. An opaque layer need not block (i.e. reflect or absorb) 100 percent of the light from an excitation beam. In some embodiments, an opaque layer may block at least 10 percent of incident light from an excitation beam; in other embodiments, an opaque layer may block at least 50 percent of incident light from an excitation beam.

Opaque layers or coatings may be fabricated on solid state membranes by a variety of techniques known in the art. Material deposition techniques may be used including chemical vapor deposition, electrodeposition, epitaxy, thermal oxidation, physical vapor deposition, including evaporation and sputtering, casting, and the like. In some embodiments, atomic layer deposition may be used, e.g. U.S. patent 6,464,842; Wei et al, Small, 6(13): 1406-1414 (2010).

### Labels for Nanopores and Analytes

In some embodiments, a nanopore may be labeled with one or more quantum dots. In particular, in some embodiments, one or more quantum dots may be attached to a nanopore, or attached to a solid phase support adjacent to (and within a FRET distance of an entrance or exit of a nanopore), and employed as donors in FRET reactions with acceptors on analytes. Such uses of quantum dots are well known and are described widely in the scientific and patent literature, such as, in U.S. patents 6,252,303; 6,855,551; 7,235,361; and the like.

One example of a Quantum dot which may be utilized as a pore label is a CdTe quantum dot which can be synthesized in an aqueous solution. A CdTe quantum dot may be functionalized with a nucleophilic group such as primary amines, thiols or functional groups such as carboxylic acids. A CdTe quantum dot may include a mercaptopropionic acid capping ligand, which has a carboxylic acid functional group that may be utilized to covalently link a quantum dot to a primary amine on the exterior of a protein pore. The cross-linking reaction may be accomplished using standard cross-linking reagents (homo-bifunctional as well as hetero-bifunctional) which are known to those having ordinary skill in the art of bioconjugation. Care may be taken to ensure that the modifications do not impair or substantially impair the translocation of a nucleic acid through the nanopore. This may be achieved by varying the length of the employed crosslinker molecule used to attach the donor label to the nanopore.

For example, the primary amine of the lysine residue 131 of the natural alpha hemolysin protein (Song, L. et al., Science 274, (1996): 1859-1866) may be used to covalently bind carboxy modified CdTe Quantum dots via 1-Ethyl-3-[3-dimethylaminopropyl]carbodiimide hydrochloride/ N-hydroxysulfosuccinimide (EDC/NHS) coupling chemistry. Alternatively, amino acid 129 (threonine) may be exchanged into cysteine. Since there is no other cysteine residue in the natural alpha hemolysin protein the thiol side group of the newly inserted cysteine may be used to covalently attach other chemical moieties.

A biological polymer, e.g., a nucleic acid molecule or polymer, may be labeled with one or more acceptor labels. For a nucleic acid molecule, each of the four nucleotides or building blocks of a nucleic acid molecule may be labeled with an acceptor label thereby creating a labeled (e.g., fluorescent) counterpart to each naturally occurring nucleotide. The acceptor label may be in the form of an energy accepting molecule which can be attached to one or more nucleotides on a portion or on the entire strand of a converted nucleic acid.

A variety of methods may be utilized to label the monomers or nucleotides of a nucleic acid molecule or polymer. A labeled nucleotide may be incorporated into a nucleic acid during synthesis of a new nucleic acid using the original sample as a template ("labeling by synthesis"). For example, the labeling of nucleic acid may be achieved via PCR, whole genome amplification, rolling circle amplification, primer extension or the like or via various combinations and extensions of the above methods known to persons having ordinary skill in the art.

A label may comprise a reactive group such as a nucleophile (amines, thiols etc.). Such nucleophiles, which are not present in natural nucleic acids, can then be used to attach fluorescent labels via amine or thiol reactive chemistry such as NHS esters, maleimides, epoxy rings, isocyanates etc. Such nucleophile reactive fluorescent dyes (i.e. NHS-dyes) are readily commercially available from different sources. An advantage of labeling a nucleic acid with small nucleophiles lies in the high efficiency of incorporation of such labeled nucleotides when a "labeling by synthesis" approach is used. Bulky fluorescently labeled nucleic acid building blocks may be poorly incorporated by polymerases due to steric hindrance of the labels during the polymerization process into newly synthesized DNA.

Whenever two or more mutually quenching dyes are used, such dyes may be attached to DNA using orthogonal attachment chemistries. For example, NHS esters can be used to react very specifically with primary amines or maleimides will react with thiol groups. Either primary amines (NH₂) or thiol (SH) modified nucleotides are commercially available. These relatively small modifications are readily incorporated in a polymerase mediated DNA synthesis and can be used for subsequent labeling reactions using either NHS or maleimide modified dyes. Guidance for selecting and using such orthogonal linker chemistries may be found in Hermanson (cited above).

Additional orthogonal attachment chemistries for typical attachment positions include Huisgen-type cycloaddition for a copper-catalyzed reaction and an uncatalyzed reaction; alkene plus nitrile oxide cycloaddition, e.g. as disclosed in Gutsmiedl et al, Org. Lett., 11: 2405-2408 (2009); Diels-Alder cycloaddition, e.g. disclosed in Seelig et al, Tetrahedron Lett., 38: 7729-7732 (1997); carbonyl ligation, e.g. as disclosed in Casi et al, J. Am. Chem. Soc., 134: 5887-5892 (2012); Shao et al J. Am. Chem. Soc., 117: 3893-3899 (1995); Rideout, Science, 233: 561-563 (1986); Michael addition, e.g. disclosed in Brinkley, Bioconjugate Chemistry, 3: 2-13 (1992); native chemical ligation, e.g. disclosed in Schuler et al, Bioconjugate Chemistry, 13: 1039-1043 (2002); Dawson et al, Science, 266: 776-779 (1994); or amide formation via an active ester, e.g. disclosed in Hermanson (cited above).

A combination of 1, 2, 3 or 4 nucleotides in a nucleic acid strand may be exchanged with their labeled counterpart. The various combinations of labeled nucleotides can be sequenced in parallel, e.g., labeling a source nucleic acid or DNA with combinations of 2 labeled nucleotides in addition to the four single labeled samples, which will result in a total of 10 differently labeled sample nucleic acid molecules or DNAs (G, A, T, C, GA, GT, GC, AT, AC, TC). The resulting sequence pattern may allow for a more accurate sequence alignment due to overlapping nucleotide positions in the redundant sequence read- out. In some embodiments, a polymer, such as a polynucleotide or polypeptide, may be labeled with a single fluorescent label attached to a single kind of monomer, for example, every T (or substantially every T) of a polynucleotide is labeled with a fluorescent label, e.g. a cyanine dye. In such embodiments, a collection, or sequence, of fluorescent signals from the polymer may form a signature or fingerprint for the particular polymer. In some such embodiments, such fingerprints may or may not provide enough information for a sequence of monomers to be determined.

In some embodiments, a feature of the invention is the labeling of substantially all monomers of a polymer analyte with fluorescent dyes or labels that are members of a mutually quenching set. The use of the term "substantially all" in reference to labeling polymer analytes is to acknowledge that chemical and enzymatic labeling techniques are typically less than 100 percent efficient. In some embodiments, "substantially all" means at least 80 percent of all monomer have fluorescent labels attached. In other embodiments, "substantially all" means at least 90 percent of all monomer have fluorescent labels attached. In other embodiments, "substantially all" means at least 95 percent of all monomer have fluorescent labels attached.

A method for sequencing a polymer, such as a nucleic acid molecule includes providing a nanopore or pore protein (or a synthetic pore) inserted in a membrane or membrane like structure or other substrate. The base or other portion of the pore may be modified with one or more pore labels. The base may refer to the Trans side of the pore. Optionally, the Cis and/or Trans side of the pore may be modified with one or more pore labels. Nucleic acid polymers to be analyzed or sequenced may be used as a template for producing a labeled version of the nucleic acid polymer, in which one of the four nucleotides or up to all four nucleotides in the resulting polymer is/are replaced with the nucleotide's labeled analogue(s). An electric field is applied to the nanopore which forces the labeled nucleic acid polymer through the nanopore, while an external monochromatic or other light source may be used to illuminate the nanopore, thereby exciting the pore label. As, after or before labeled nucleotides of the nucleic acid pass through, exit or enter the nanopore, energy is transferred from the pore label to a nucleotide label, which results in emission of lower energy radiation. The nucleotide label radiation is then detected by a confocal microscope setup or other optical detection system or light microscopy system capable of single molecule detection known to people having ordinary skill in the art. Examples of such detection systems include but are not limited to confocal microscopy, epifluorescent microscopy and total internal reflection fluorescent (TIRF) microscopy. Other polymers (e.g., proteins and polymers other than nucleic acids) having labeled monomers may also be sequenced according to the methods described herein. In some embodiments, fluorescent labels or donor molecules are excited in a TIRF system with an evanescent wave, sometimes referred to herein as "evanescent wave excitation."

Energy may be transferred from a pore or nanopore donor label (e.g., a Quantum Dot) to an acceptor label on a polymer (e.g., a nucleic acid) when an acceptor label of an acceptor labeled monomer (e.g., nucleotide) of the polymer interacts with the donor label as, after or before the labeled monomer exits, enters or passes through a nanopore. For example, the donor label may be positioned on or attached to the nanopore on the cis or trans side or surface of the nanopore such that the interaction or energy transfer between the donor label and acceptor label does not take place until the labeled monomer exits the nanopore and comes into the vicinity or proximity of the donor label outside of the nanopore channel or opening. As a result, interaction between the labels, energy transfer from the donor label to the acceptor label, emission of energy from the acceptor label and/or measurement or detection of an emission of energy from the acceptor label may take place outside of the passage, channel or opening running through the nanopore, e.g., within a cis or trans chamber on the cis or trans sides of a nanopore. The measurement or detection of the energy emitted from the acceptor label of a monomer may be utilized to identify the monomer.

The nanopore label may be positioned outside of the passage, channel or opening of the nanopore such that the label may be visible or exposed to facilitate excitation or illumination of the label. The interaction and energy transfer between a donor label and accepter label and the emission of energy from the acceptor label as a result of the energy transfer may take place outside of the passage, channel or opening of the nanopore. This may facilitate ease and accuracy of the detection or measurement of energy or light emission from the acceptor label, e.g., via an optical detection or measurement device.

A donor label may be attached in various manners and/or at various sites on a nanopore. For example, a donor label may be directly or indirectly attached or connected to a portion or unit of the nanopore. Alternatively, a donor label may be positioned adjacent to a nanopore.

Each acceptor labeled monomer (e.g., nucleotide) of a polymer (e.g., nucleic acid) can interact sequentially with a donor label positioned on or next to or attached directly or indirectly to the exit of a nanopore or channel through which the polymer is translocated. The interaction between the donor and acceptor labels may take place outside of the nanopore channel or opening, e.g., after the acceptor labeled monomer exits the nanopore or before the monomer enters the nanopore. The interaction may take place within or partially within the nanopore channel or opening, e.g., while the acceptor labeled monomer passes through, enters or exits the nanopore.

When one of the four nucleotides of a nucleic acid is labeled, the time dependent signal arising from the single nucleotide label emission is converted into a sequence corresponding to the positions of the labeled nucleotide in the nucleic acid sequence. The process is then repeated for each of the four nucleotides in separate samples and the four partial sequences are then aligned to assemble an entire nucleic acid sequence.

When multi-color labeled nucleic acid (DNA) sequences are analyzed, the energy transfer from one or more donor labels to each of the four distinct acceptor labels that may exist on a nucleic acid molecule may result in light emission at four distinct wavelengths or colors (each associated with one of the four nucleotides) which allows for a direct sequence read-out.

A donor label (also sometimes referred to herein as a "pore label") may be placed as close as possible to the aperture (for example, at the exit) of a nanopore without causing an occlusion that impairs translocation of a nucleic acid through the nanopore. A pore label may have a variety of suitable properties and/or characteristics. For example, a pore label may have energy absorption properties meeting particular requirements. A pore label may have a large radiation energy absorption cross-section, ranging, for example, from about 0 to 1000 nm or from about 200 to 500 nm. A pore label may absorb radiation within a specific energy range that is higher than the energy absorption of the nucleic acid label, such as an acceptor label. The absorption energy of the pore label may be tuned with respect to the absorption energy of a nucleic acid label in order to control the distance at which energy transfer may occur between the two labels. A pore label may be stable and functional for at least 106 to 109 excitation and energy transfer cycles.

Described herein is a device for analyzing polymers each having optical labels attached to a sequence of monomers may comprise the following elements: (a) a nanopore array in a solid phase membrane separating a first chamber and a second chamber, wherein nanopores of the nanopore array each provide fluid communication between the first chamber and the second chamber and are arranged in clusters such that each different cluster of nanopores is disposed within a different resolution limited area and such that each cluster comprises a number of nanopores that is either greater than one or is a random variable with an average value greater than zero; (b) a polymer translocating system for moving polymers in the first chamber to the second chamber through the nanopores of the nanopore array; and (c) a detection system for collecting optical signals generated by optical labels attached to polymers whenever an optical label exits a nanopore within a resolution limited area.

### Two-Color Sequencing

In some embodiments, the invention is used in two-color optically based nanopore sequencing. Optically based nanopore sequencing may be implemented with as few as two different kinds of nucleotide being labeled with different optical labels. In such approaches, the two optical labels generate distinguishable optical signals for the selected kinds of nucleotide in both sense strands and antisense strands of target polynucleotides. For example, C's and T's of the complementary strands of each target polynucleotide may be replaced by labeled analogs, wherein the labels of the C and T analogs are capable of generating distinct optical signals. Optical signatures are then generated by translocating the labeled strands through nanopores where nucleotides of the strands are constrained to pass sequentially through an optical detection region where their labels are caused to generate optical signals. Information from optical signatures from both sense and antisense strands are combined to determine a nucleotide sequence of target polynucleotides.

In some embodiments, the selected kinds of nucleotides of target polynucleotides are replaced by labeled nucleotide analogs in an extension reaction using a nucleic acid polymerase. Labeled strands of target polynucleotides are translocated through nanopores that constrain the nucleotides of strands to move single file through an optical detection region where they are excited so that they produce an optical signal. A collection of optical signals for an individual strand is referred to herein as an optical signature of the strand. In some embodiments, where a strand and its complement (i.e. sense and antisense strands) are linked, for example, via a hairpin adaptor, a single optical signature may include optical signals from optical labels on nucleotides from both the sense strand and the antisense strand. In other embodiments, different strands of a target polynucleotide may separately generate two different optical signatures which may be combined, or used together, for analysis, as mentioned above. Such separately analyzed strands may be associated after generation of optical signatures, for example, by using molecular tags (which may be, for example, oligonucleotide segments attached to target polynucleotides in a known position, length and sequence pattern and diversity to permit ready association). An optical signature may comprise mixed optical signals in that the signal detected in each detection interval may comprise contributions from multiple optical labels emitting within a resolution limited area or volume; that is, they may (for example) be mixed FRET signals, as described by Huber et al, U.S. patent publication US20160076091.

In some embodiments, such two-color optically based nanopore sequencing may be implemented with the following steps: (a) copying a strand of a double stranded polynucleotide by extending a primer having a 5' non-complementary tail on the strand to produce a double stranded product comprising an a labeled strand with the 5' non-complementary tail as a single stranded overhang, wherein nucleotide analogs with distinct optical labels are substituted for at least two kinds of nucleotide to form a labeled strand; (b) copying a complement of the strand by extending a primer having a 5' non-complementary tail on the complement of the strand to produce a double stranded product comprising an a labeled complement with the 5' non-complementary tail as a single stranded overhang, wherein nucleotide analogs with distinct optical labels are substituted for at least two kinds of nucleotide to form a labeled complement; (c) capturing by a nanopore the 5' non-complementary tail of the double stranded product comprising the labeled strand by applying an electrical field across the nanopore; (d) translocating the labeled stand through a nanopore so that the nucleotides of the labeled strand pass single file through an excitation zone at a rate of less than 1000 nucleotides per second (nt/sec), wherein optical labels are excited to generate optical signals and wherein the translocating strand of the double stranded product is unzipped as it enters the nanopore; (e) detecting a time series of optical signals from the optical labels as the labeled strand translocates through the nanopore to produce a strand optical signature; (f) capturing by a nanopore the 5' non-complementary tail of the double stranded product comprising the labeled complement by applying an electrical field across the nanopore, (g) translocating the labeled complement through a nanopore at a rate of less than 1000 nucleotides per second (nt/sec), wherein optical labels are excited to generate optical signals and wherein the translocating strand of the double stranded product is unzipped as it enters the nanopore; (h) detecting a time series of optical signals from the optical labels as the labeled complement translocates through the nanopore to produce a complement optical signature; (i) determining a sequence of the double stranded polynucleotide from the strand optical signature and the complement optical signature.

In some embodiments, two kinds of nucleotide are labeled, which may be C's and T's, C's and G's, C's and A's, T's and G's, T's and A's, or G's and A's. In some embodiments, pyrimidine nucleotides are labeled. In other embodiments, purine nucleotides are labeled. In some embodiments, selected kinds of nucleotides of a strand are labeled by incorporating labeled analog dNTPs of the selected kind of nucleotides in a primer extension reaction using a nucleic acid polymerase. In other embodiments, selected kinds of nucleotides of a strand are labeled by incorporating analog dNTPs of the selected kinds of nucleotides in an extension reaction, wherein the analog dNTPs are derivatized with orthogonally reactive functionalities that allow attachment of different labels to different kinds of nucleotides in a subsequent reaction. This latter labeling approach is disclosed in Jett et al, U.S. patent 5,405,747.

In some embodiments, three kinds of nucleotide are labeled, which may include labeling C's with a first optical label, T's with a second optical label, and G's and A's with a third optical label. In other embodiments, the following groups of nucleotides may be labeled as indicated: C's and G's with a first optical label and second optical label, respectively, and T's and A's with a third optical label; C's and A's with a first optical label and second optical label, respectively, and T's and G's with a third optical label; T's and G's with a first optical label and second optical label, respectively, and C's and A's with a third optical label; A's and G's with a first optical label and second optical label, respectively, and T's and C's with a third optical label.

In some embodiments, optical labels are fluorescent acceptor molecules that generate a fluorescent resonance energy transfer (FRET) signal after energy transfer from a donor associated with a nanopore. In some embodiments, as described further below, donors may be optically active nanoparticles, such as, quantum dots, nanodiamonds, or the like. Selection of particular combinations of acceptor molecules and donors are design choices for one of ordinary skill in the art. In some embodiments, some of which are described more fully below, a single quantum dot is attached to a nanopore and is excited to fluoresce using an excitation beam whose wavelength is sufficiently separated, usually lower (i.e. bluer), so that it does not contribute to FRET signals generated by acceptors. Likewise, a quantum dot is selected whose emission wavelength overlaps the absorption bands of both acceptor molecules to facilitate FRET interactions. In some embodiments, two donors may be used for each excitation zone of a nanopore, wherein the emission wavelength of each is selected to optimally overlap the absorption band of a different one of the acceptor molecules.

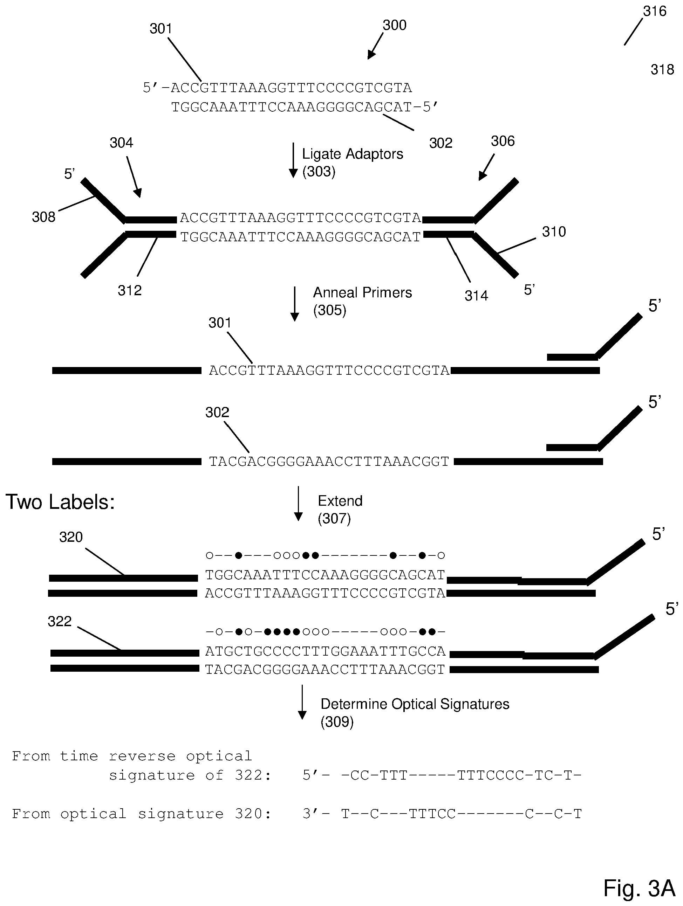

In Fig. 3A, double stranded target polynucleotide (300) consists of sense strand (301) and complementary antisense strand (302), to which is ligated (303) "Y" adaptors (304) and (306) using conventional methods, e.g. Weissman et al, U.S. patent 6,287,825; Schmitt et al, U.S. patent publication US2015/004468. Arms (308) and (310) of adaptors (304 and 306, respectively) include primer binding sites to which primers (316) and (318) are annealed (305). Double stranded portions (312) and (314) may include tag sequences, e.g. one or both may include randomers of predetermined length and composition, which may be used for later re-association of the strands, for example, to obtain sequence information from the respective optical signatures of the strands. After annealing primers (316) and (318), they may be extended (307) by a nucleic acid polymerase in the presence of (for example, as illustrated) labeled dUTP analogs (labels shown as open circles in the incorporated nucleotides) and labeled dCTP analogs (labels shown as filled circles in the incorporated nucleotides) and natural unlabeled dGTPs and dATPs (with neither unlabeled dTTP nor unlabeled dCTP being present so that the analogs are fully substituted in the extended strands). The absence of labels on G's and A's are illustrated as dashes above the incorporated nucleotides. In an ideal detection system without noise, the sequence of open circles, filled circles and dashes would be good representations of optical signatures generated by the indicated sense and antisense strands as they pass through an excitation zone of a nanopore.

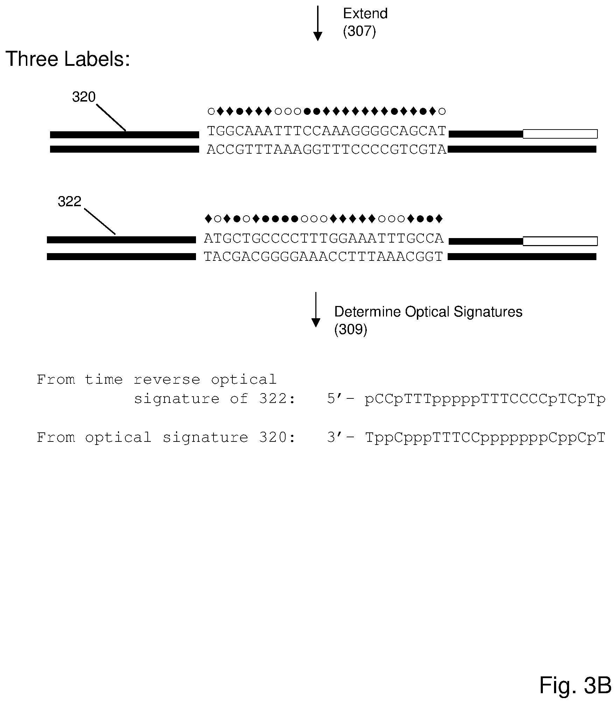

In Fig. 3B, extension products (320) and (322) are illustrated for an alternative embodiment employing three labels. Incorporated labeled dUTP analogs are shown as open circles and incorporated labeled dCTP analogs are shown as filled circles, as above. Incorporated labeled dATP and dGTP analogs are shown as filled diamonds.

Guidance in selecting the kinds of nucleotide to label, kinds of labels and linkers for attaching them to bases, and nucleic acid polymerases for extension reactions in the presence of dNTP analogs can be found in the following references: Goodman et al, U.S. patent 5,945,312; Jett et al, U.S. patent 5,405,747; Muehlegger et al, U.S. patent publication US2004/0214221; Giller et al, Nucleic Acids Research, 31(10): 2630-2635 (2003); Tasara et al, Nucleic Acids Research, 31(10): 2636-2646 (2003); Augustin et al, J. Biotechnology, 86: 289-301 (2001); Brakmann, Current Pharmacuetical Biotechnology, 5(1): 119-126 (2004); and the like. Exemplary nucleic acid polymerases for use with the invention include, but are not limited to, Vent exo⁻, Taq, E. coli Pol I, Tgo exo⁻, Klenow fragment exo⁻, Deep Vent exo⁻, and the like. In some embodiments, exemplary nucleic acid polymerases include, but are not limited to, Vent exo⁻ and Klenow fragment exo⁻. Exemplary fluorescent labels for dNTP analogs include, but are not limited to, Alexa 488, AMCA, Atto 655, Cy3, Cy5, Evoblue 30, fluorescein, Gnothis blue 1, Gnothis blue 2, Gnothis blue 3, Dy630, Dy635, MR121, rhodamine, Rhodamine Green, Oregon Green, TAMRA, and the like. Exemplary fluorescent labels for dUTP analogs include, but are not limited to, Alexa 488, AMCA, Atto 655, Cy3, Cy5, Dy630, Dy665, Evoblue 30, Evoblue 90, fluorescein, Gnothis blue 1, Gnothis blue 2, Gnothis blue 3, MR121, Oregon Green, rhodamine, Rhodamine Green, TAMRA, and the like. Exemplary fluorescent labels for dCTP analogs include, but are not limited to, Atto 655, Cy5, Evoblue 30, Gnothis blue 3, rhodamine, Rhodamine Green, TAMRA, and the like. Exemplary fluorescent labels for dATP analogs include, but are not limited to, Atto 655, Cy5, Evoblue 30, Gnothis blue 3, Rhodamine Green, and the like. Exemplary fluorescent labels for dGTP analogs include, but are not limited to, Evoblue 30, Gnothis blue 3, Rhodamine Green, and the like. Exemplary pairs of fluorescent labels for dUTP analogs and dCTP analogs include, but are not limited to, (TAMRA, Rhodamine Green), (Atto 655, Evoblue 30), (Evoblue 30, Atto 655), (Evoblue 30, Gnothis blue 3), (Evoblue 30, Rhodamine Green), (Gnothis blue 1, Rhodamine Green), (Gnothis blue 2, Atto 655), Gnothis blue 3, Cy5), and the like.

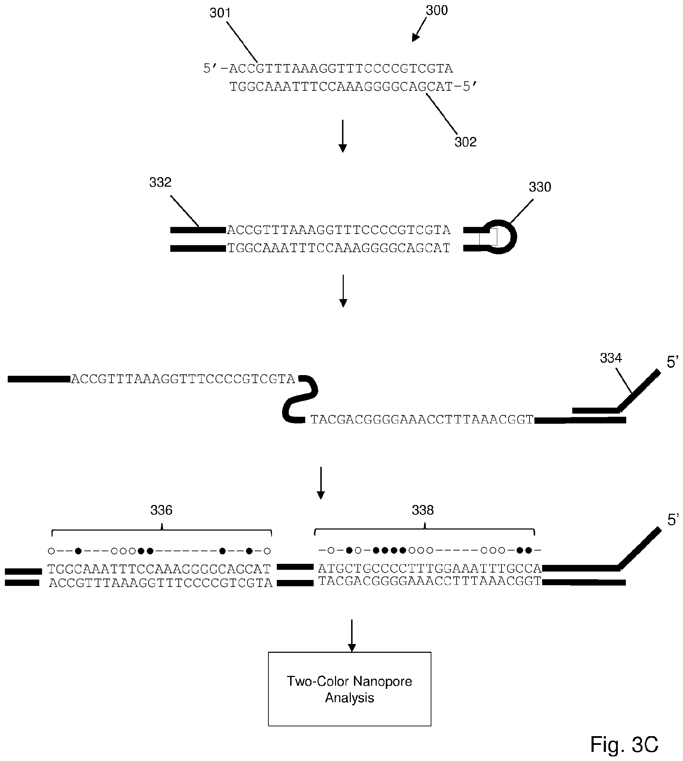

Fig. 3C illustrates an embodiment in which two labels are used and sense and antisense strands are linked by means of hairpin adaptor (330), for example, as taught in U.S. patent publications US 2015/0152492 and US 2012/0058468. Tailed adaptor (332) and hairpin adaptor (330) are ligated to target polynucleotide (300). After denaturation and annealing of primer (334), an extension reaction produces extension product (335) which includes segment (336), the labeled complement of strand (301) and segment (338), the labeled reverse complement of strand (301). After translocation of extension product (335) through a nanopore and generation of an optical signature the sequence of target polynucleotide (300) can be determined. Optionally, the sequence of hairpin (330) may be selected so that a predetermined pattern of labels is incorporated during the extension reaction, which may be used to assist in the analysis of the optical signature, e.g. by indicating where segment (336) ends and where segment (338) begins, or the like.

### Example

### Translocation of Target Polynucleotide in An

### Optically-Based Nanopore Sequencing Method

In this example, the invention is used in conjunction with an exemplary optically-based nanopore sequencing method. In the exemplary optically-based nanopore sequencing method, nucleotides of target polynucleotides are labeled with fluorescent labels that are capable of at least three states: (i) A quenched state wherein fluorescence of an attached fluorescent label is quenched by a fluorescent label on an immediately adjacent nucleotide; for example, a fluorescent label attached to a polynucleotide is quenched when the labeled polynucleotide is free in an aqueous solution. (ii) A sterically constrained state wherein a labeled polynucleotide is translocating through a nanopore such that the free-solution movements or alignments of an attached fluorescent label is disrupted or limited so that there is little or no detectable signal generated from the fluorescent label. (iii) A transition state wherein a fluorescent label attached to a polynucleotide transitions from the sterically constrained state to the quenched state as the fluorescent label exits the nanopore (during a "transition interval") while the polynucleotide translocates through the nanopore. A nucleotide sequence of a polynucleotide is determined by recording signals generated by attached fluorescent labels as they exit a nanopore one at a time as a polynucleotide translocates the nanopore. Upon exit, each attached fluorescent label transitions during a transition interval from a constrained state in the nanopore to a quenched state on the polynucleotide in free solution. During this transition interval the fluorescent label is capable of emitting a detectable fluorescent signal indicative of the nucleotide it is attached to.

Described herein is a nanopore sequencing method using the following steps: (a) extending a primer having a 5' non-complementary tail on a template in a reaction mixture to produce a double stranded product comprising an extended strand and the 5' non-complementary tail as a single stranded overhang; (b) providing a nanopore (or an array of nanopores) that separates and provides fluid communication between a first chamber and a second chamber, wherein the nanopore is capable of passing a single stranded nucleic acid but not a double stranded nucleic acid; (c) disposing the double stranded product in the first chamber; (d) capturing the 5' non-complementary tail of the isolated double stranded product by the nanopore by applying an electrical field across the nanopore; (e) translocating a polymer analyte through a nanopore having a bore and an exit, the polymer analyte comprising a sequence of monomers, wherein substantially each monomer is labeled with a fluorescent label such that fluorescent labels of adjacent monomers are in a quenched state by self-quenching one another outside of the nanopore and fluorescent labels are in a sterically constrained state and incapable of generating a detectable fluorescent signal inside of the nanopore; (f) exciting each fluorescent label at the exit of the nanopore as it transitions from a sterically constrained state to a quenched state so that a fluorescent signal is generated which is indicative of the monomer to which it is attached; (g) detecting the fluorescent signal to identify the monomer. As used herein, "substantially every", "substantially all", or like terms, in reference to labeling monomers, particularly nucleotides, acknowledges that chemical labeling procedures may not result in complete labeling of every monomer; to the extent practicable, the terms comprehend that labeling reactions in connection with the invention are continued to completion; in some embodiments, such completed labeling reactions include labeling at least fifty percent of the monomers; in other embodiments, such labeling reactions include labeling at least eighty percent of the monomers; in other embodiments, such labeling reactions include labeling at least ninety-five percent of the monomers; in other embodiments, such labeling reactions include labeling at least ninety-nine percent of the monomers.

In some embodiments, a nucleotide sequence of a target polynucleotide is determined by carrying out four separate reactions in which copies of the target polynucleotide have each of its four different kinds of nucleotide (A, C, G and T) labeled with a single fluorescent label. In a variant of such embodiments, a nucleotide sequence of a target polynucleotide is determined by carrying out four separate reactions in which copies of the target polynucleotide have each of its four different kinds of nucleotide (A, C, G and T) labeled with one fluorescent label while at the same time the other nucleotides on the same target polynucleotide are labeled with a second fluorescent label. For example, if a first fluorescent label is attached to A's of the target polynucleotide in a first reaction, then a second fluorescent label is attached to C's, G's and T's (i.e. to the "not-A" nucleotides) of the target polynucleotides in the first reaction. Likewise, in continuance of the example, in a second reaction, the first label is attached to C's of the target polynucleotide and the second fluorescent label is attached to A's, G's and T's (i.e. to the "not-C" nucleotides) of the target polynucleotide. And so on, for nucleotides G and T.

The same labeling scheme may be expressed in terms of conventional terminology for subsets of nucleotide types; thus, in the above example, in a first reaction, a first fluorescent label is attached to A's and a second fluorescent label is attached to B's; in a second reaction, a first fluorescent label is attached to C's and a second fluorescent label is attached to D's; in a third reaction, a first fluorescent label is attached to G's and a second fluorescent label is attached to H's; and in a fourth reaction, a first fluorescent label is attached to T's and a second fluorescent label is attached to V's.

In some embodiments, a feature of the sequencing method is the labeling of substantially all monomers of a polymer analytes with fluorescent dyes or labels that are members of a mutually quenching set. Such sets of fluorescent dyes have the following properties: (i) each member quenches fluorescence of every member (for example, by FRET or by static or contact mechanisms), and (ii) each member generates a distinct fluorescent signal when excited and when in a non-quenching state. That is, if a mutually quenching set consists of two dyes, D1 and D2, then (i) D1 is self-quenched (e.g. by contact quenching with another D1 molecule) and it is quenched by D2 (e.g. by contact quenching) and (ii) D2 is self-quenched (e.g. by contact quenching with another D2 molecule) and it is quenched by D1 (e.g. by contact quenching). Guidance for selecting fluorescent dyes or labels for mutually quenching sets may be found in the following references:Johansson, Methods in Molecular Biology, 335: 17-29 (2006); Marras et al, Nucleic Acids Research, 30: e122 (2002); and the like. Exemplary mutually quenching sets of fluorescent dyes, or labels, may be selected from rhodamine dyes, fluorescein dyes and cyanine dyes. In one embodiment, a mutually quenching set may comprise the rhodamine dye, TAMRA, and the fluorescein dye, FAM. In another embodiment, mutually quenching sets of fluorescent dyes may be formed by selecting two or more dyes from the group consisting of Oregon Green 488, Fluorescein-EX, fluorescein isothiocyanate, Rhodamine Red-X, Lissamine rhodamine B, Calcein, Fluorescein, Rhodamine, one or more BODIPY dyes, Texas Red, Oregon Green 514, and one or more Alexa Fluors. Representative BODIPY dyes include BODIPY FL, BODIPY R6G, BODIPY TMR, BODIPY 581/591, BODIPY TR, BODIPY 630/650 and BODIPY 650/665. Representative Alexa Fluors include Alexa Fluor 350, 405, 430, 488, 500, 514, 532, 546, 555, 568, 594, 610, 633, 635, 647, 660, 680, 700, 750 and 790.

In some embodiments of the above method, fluorescent labels are members of a FRET pair. A FRET pair generally is one or more FRET donors and one or more FRET acceptors where each donor is capable of a FRET reaction with each acceptor. In one aspect, this means that the donors of the FRET pair have an emission spectrum that substantially overlaps the absorption spectrum of the acceptors. In another aspect, the transition dipole of the donor and the acceptor have to be aligned in a way that allows efficient energy transfer. In some aspects, the invention in part is based on the discovery and appreciation of a fluorescence, particularly, FRET suppressing property of nanopores and the application of this property to enable detection of labeled polymers translocating through a nanopore. It is believed, although the invention is not intended to be limited thereby, that a nanopore may be selected with a bore dimensioned so that a FRET pair label cannot orient to engage in a FRET interaction while translocating through the nanopore. The dipoles of the labels of the polynucleotide in the bore of the nanopore are constrained in their rotational freedom based on the limited diameter of the nanopore. This reduction in dipole alignment with the alignment of the corresponding FRET pair attached to the nanopore limits the FRET efficiency dramatically. Labeled polynucleotides can engage in a FRET interaction after exiting the nanopore at which point the FRET acceptor or donor on the polymer (e.g. polynucleotide) regains rotational freedom which allows for a FRET event.

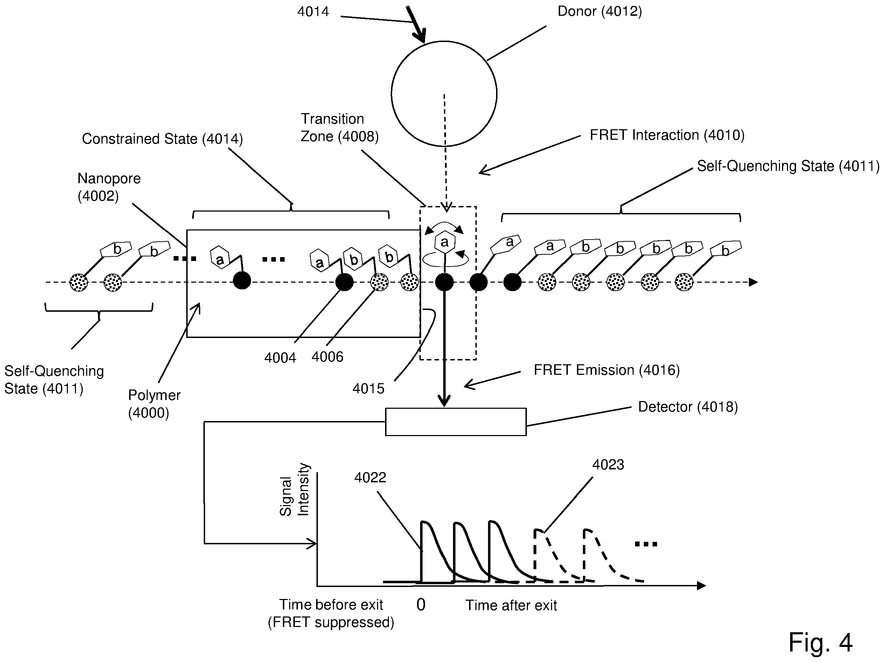

Some of the above aspects and embodiments of the above method are illustrated diagrammatically in Fig. 4. Polymer analyte (4000), such as a polynucleotide, is captured by then driven, e.g. electrophoretically, through nanopore (4002), which constrains the conformation of polymer (4000) so that its monomeric units translocate through the nanopore in the same order as their primary sequence in the polymer. In Fig. 4, fluorescent labels are assumed to be members of FRET pairs; fluorescent labels may also include fluorescent labels that are directly excited, for example with a laser emitting at an appropriate wavelength, to generate a fluorescent signal.

As mentioned above, whenever an acceptor-labeled monomeric unit is within the bore of nanopore (4002), FRET interactions between such acceptors and the donors of its FRET pair are suppressed because acceptors are in a constrained state (4014). Such suppression typically means that no detectable FRET signal is produced even if such acceptors are within a FRET distance of a donor, for example, due to unfavorable orientation of the acceptor and donor dipoles. On the other hand, when an acceptor-labeled monomeric unit emerges from the bore of, or exits, the nanopore into transition zone (4008), FRET interaction (4010) occurs and FRET emission (4016) is produced and detected by detector (4018) until the acceptor enters a self-quenching state (4011) with an adjacent acceptor and as the distance between the acceptor and donor increases with the movement of polymer (4000) out of FRET interaction distance. Signal (4022) is produced by a single acceptor as it moves through transition zone (4008). Transition zone (4008), which is a spatial region immediately adjacent to exit (4015) of nanopore (4002), is defined by several factors, including the speed of the translocation of polymer (4000) through nanopore (4002), the vibrational and rotational mobility of the fluorescent labels, the physiochemical nature of the fluorescent labels, and the like. In Fig. 4, only one type of monomeric unit, illustrated as solid circles (4004) carries a first fluorescent label (designated as "a"); the rest of the monomeric units, illustrated as speckled circles (4006), carry a second fluorescent label (designated as "b"). In this embodiment, first fluorescent labels quench adjacent first fluorescent labels and adjacent second fluorescent labels; likewise, second fluorescent labels quench adjacent first fluorescent labels and adjacent second fluorescent labels; moreover, the first and second fluorescent labels generate FRET signals that are distinguishable from one another, for example, recorded signal (4022) for label "a" and recorded signal (4023) for label "b" in Fig. 4, so that each fluorescent label (and hence, monomer) may be identified by a signal detected by detector (4018).

### Definitions

"FRET" or "Forster, or fluorescence, resonant energy transfer" means a non-radiative dipole-dipole energy transfer mechanism from an excited donor fluorophore to an acceptor fluorophore in a ground state. The rate of energy transfer in a FRET interaction depends on the extent of spectral overlap of the emission spectrum of the donor with the absorption spectrum of the acceptor, the quantum yield of the donor, the relative orientation of the donor and acceptor transition dipoles, and the distance between the donor and acceptor molecules, Lakowitz, Principles of Fluorescence Spectroscopy, Third Edition (Springer, 2006). FRET interactions of particular interest are those which result a portion of the energy being transferred to an acceptor, in turn, being emitted by the acceptor as a photon, with a frequency lower than that of the light exciting its donor (i.e. a "FRET signal"). "FRET distance" means a distance between a FRET donor and a FRET acceptor over which a FRET interaction can take place and a detectable FRET signal produced by the FRET acceptor.

"Kit" refers to any delivery system for delivering materials or reagents for carrying out a method of the invention. In the context of reaction assays, such delivery systems include systems that allow for the storage, transport, or delivery of reaction reagents (e.g., fluorescent labels, such as mutually quenching fluorescent labels, fluorescent label linking agents, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. Such contents may be delivered to the intended recipient together or separately. For example, a first container may contain an enzyme for use in an assay, while a second or more containers contain mutually quenching fluorescent labels.

"Nanopore" means any opening positioned in a substrate that allows the passage of analytes through the substrate in a predetermined or discernable order, or in the case of polymer analytes, passage of their monomeric units through the substrate in a predetermined or discernible order. In the latter case, a predetermined or discernible order may be the primary sequence of monomeric units in the polymer. Examples of nanopores include proteinaceous or protein based nanopores, synthetic or solid state nanopores, and hybrid nanopores comprising a solid state nanopore having a protein nanopore embedded therein. A nanopore may have an inner diameter of 1-10 nm or 1-5 nm or 1-3 nm. Examples of protein nanopores include but are not limited to, alpha-hemolysin, voltage-dependent mitochondrial porin (VDAC), OmpF, OmpC, MspA and LamB (maltoporin), e.g. disclosed in Rhee, M. et al., Trends in Biotechnology, 25(4) (2007): 174-181; Bayley et al (cited above); Gundlach et al, U.S. patent publication 2012/0055792; and the like. Any protein pore that allows the translocation of single nucleic acid molecules may be employed. A nanopore protein may be labeled at a specific site on the exterior of the pore, or at a specific site on the exterior of one or more monomer units making up the pore forming protein. Pore proteins are chosen from a group of proteins such as, but not limited to, alpha-hemolysin, MspA, voltage-dependent mitochondrial porin (VDAC), Anthrax porin, OmpF, OmpC and LamB (maltoporin). Integration of the pore protein into the solid state hole is accomplished by attaching a charged polymer to the pore protein. After applying an electric field the charged complex is electrophoretically pulled into the solid state hole. A synthetic nanopore, or solid-state nanopore, may be created in various forms of solid substrates, examples of which include but are not limited to silicones (e.g. Si3N4, SiO2), metals, metal oxides (e.g. Al2O3) plastics, glass, semiconductor material, and combinations thereof. A synthetic nanopore may be more stable than a biological protein pore positioned in a lipid bilayer membrane. A synthetic nanopore may also be created by using a carbon nanotube embedded in a suitable substrate such as but not limited to polymerized epoxy. Carbon nanotubes can have uniform and well-defined chemical and structural properties. Various sized carbon nanotubes can be obtained, ranging from one to hundreds of nanometers. The surface charge of a carbon nanotube is known to be about zero, and as a result, electrophoretic transport of a nucleic acid through the nanopore becomes simple and predictable (Ito, T. et al., Chem. Commun. 12 (2003): 1482-83). The substrate surface of a synthetic nanopore may be chemically modified to allow for covalent attachment of the protein pore or to render the surface properties suitable for optical nanopore sequencing. Such surface modifications can be covalent or non-covalent. Most covalent modification include an organosilane deposition for which the most common protocols are described: 1) Deposition from aqueous alcohol. This is the most facile method for preparing silylated surfaces. A 95% ethanol-5% water solution is adjusted to pH 4.5-5.5 with acetic acid. Silane is added with stirring to yield a 2% final concentration. After hydrolysis and silanol group formation the substrate is added for 2-5min. After rinsed free of excess materials by dipping briefly in ethanol. Cure of the silane layer is for 5-10min at 110 degrees Celsius. 2) Vapor Phase Deposition. Silanes can be applied to substrates under dry aprotic conditions by chemical vapor deposition methods. These methods favor monolayer deposition. In closed chamber designs, substrates are heated to sufficient temperature to achieve 5mm vapor pressure. Alternatively, vacuum can be applied until silane evaporation is observed. 3) Spin-on deposition. Spin-on applications can be made under hydrolytic conditions which favor maximum functionalization and polylayer deposition or dry conditions which favor monolayer deposition. In some embodiments, single nanopores are employed with methods of the invention. In other embodiments, a plurality of nanopores are employed. In some of the latter embodiments, a plurality of nanopores is employed as an array of nanopores, usually disposed in a planar substrate, such as a solid phase membrane. Nanopores of a nanopore array may be spaced regularly, for example, in a rectilinear pattern, or may be spaced randomly. In a preferred embodiment, nanopores are spaced regularly in a rectilinear pattern in a planar solid phase substrate.

"Nanostructure" (used interchangeably with "nanoscale structure" and "nanoscale feature") means a structure that has at least one dimension within a range of a few nanometers to several hundred nanometers, for example, from 1 to 1000 nanometers. In some applications, such range is from 2 to 500 nanometers; in other applications, such range is from 3 to 500 nanometers. The shape and geometry of nanostructures may vary widely and include, but are not limited to, nanopores, nanowells, nanoparticles, and any other convenient shapes particularly suitable for carrying out sequences of reactions. In some embodiments, nanostructures may be protein nanopores operationally associated with a solid phase membrane. Some nanostructures, such as, nanopores and nanowells, may be formed in a larger common substrate, such as a solid phase membrane, or other solid, to form arrays of nanopores or nanowells. Nanostructures of particular interest are those capable of supporting or containing a chemical, physical (e.g. FRET), enzymatic and/or binding reaction or a sequence of such reactions. In some embodiments, a nanostructure, such as a nanowell, encloses a volume that is less than one nanoliter (10×-9 liter), less than one picoliter, or less than one femtoliter. In other embodiments, each of the individual nanowells provides a volume that is less than 1000 zeptoliters, 100 zeptoliters, 80 zeptoliters, or less than 50 zeptoliters, or less than 1 zeptoliter, or even less than 100 yactoliters. In some embodiments, nanowells comprise zero mode waveguides.

"Peptide," "peptide fragment," "polypeptide," "oligopeptide," or "fragment" in reference to a peptide are used synonymously herein and refer to a compound made up of a single unbranched chain of amino acid residues linked by peptide bonds. Amino acids in a peptide or polypeptide may be derivatized with various moieties, including but not limited to, polyethylene glycol, dyes, biotin, haptens, or like moieties. The number of amino acid residues in a protein or polypeptide or peptide may vary widely; however, in some embodiments, protein or polypeptides or peptides referred to herein may have 2 from to 70 amino acid residues; and in other embodiments, they may have from 2 to 50 amino acid residues. In other embodiments, proteins or polypeptides or peptides referred to herein may have from a few tens of amino acid residues, e.g. 20, to up to a thousand or more amino acid residues, e.g. 1200. In still other embodiments, proteins, polypeptides, peptides, or fragments thereof, may have from 10 to 1000 amino acid residues; or they may have from 20 to 500 amino acid residues; or they may have from 20 to 200 amino acid residues.

"Polymer" means a plurality of monomers connected into a linear chain. Usually, polymers comprise more than one type of monomer, for example, as a polynucleotide comprising A's, C's, G's and T's, or a polypeptide comprising more than one kind of amino acid. Monomers may include without limitation nucleosides and derivatives or analogs thereof and amino acids and derivatives and analogs thereof. In some embodiments, polymers are polynucleotides, whereby nucleoside monomers are connected by phosphodiester linkages, or analogs thereof.

"Polynucleotide" or "oligonucleotide" are used interchangeably and each mean a linear polymer of nucleotide monomers. Monomers making up polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moieties, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is well within the knowledge of one of ordinary skill, especially with guidance from treatises, such as Sambrook et al, Molecular Cloning, Second Edition (Cold Spring Harbor Laboratory, New York, 1989), and like references. Likewise, the oligonucleotide and polynucleotide may refer to either a single stranded form or a double stranded form (i.e. duplexes of an oligonucleotide or polynucleotide and its respective complement). It will be clear to one of ordinary skill which form or whether both forms are intended from the context of the terms usage.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references: Dieffenbach, editor, PCR Primer: A Laboratory Manual, 2nd Edition (Cold Spring Harbor Press, New York, 2003).

"Sequence determination", "sequencing" or "determining a nucleotide sequence" or like terms in reference to polynucleotides includes determination of partial as well as full sequence information of the polynucleotide. That is, the terms include sequences of subsets of the full set of four natural nucleotides, A, C, G and T, such as, for example, a sequence of just A's and C's of a target polynucleotide. That is, the terms include the determination of the identities, ordering, and locations of one, two, three or all of the four types of nucleotides within a target polynucleotide. In some embodiments, the terms include the determination of the identities, ordering, and locations of two, three or all of the four types of nucleotides within a target polynucleotide. In some embodiments sequence determination may be accomplished by identifying the ordering and locations of a single type of nucleotide, e.g. cytosines, within the target polynucleotide "catcgc . . . " so that its sequence is represented as a binary code, e.g. "100101 . . . " representing "c-(not c)(not c)c-(not c)-c ... " and the like. In some embodiments, the terms may also include subsequences of a target polynucleotide that serve as a fingerprint for the target polynucleotide; that is, subsequences that uniquely identify a target polynucleotide, or a class of target polynucleotides, within a set of polynucleotides, e.g. all different RNA sequences expressed by a cell.

## Claims

1. A method of analyzing a nucleic acid comprising:
(a) extending a primer having a 5' non-complementary tail on a template in a reaction mixture to produce a double stranded product comprising a labeled extended strand and the 5' non-complementary tail as a single stranded overhang, wherein labels of said labeled extended strand are optical labels each capable of generating an optical signal indicative of a nucleotide to which it is attached;
(b) providing at least one nanopore that provides fluid communication between a first chamber and a second chamber, wherein each nanopore of the at least one nanopore is capable of passing a single stranded nucleic acid but not a double stranded nucleic acid;
(c) disposing the double stranded product in the first chamber;
(d) capturing by the at least one nanopore the 5' non-complementary tail of the double stranded product by applying an electrical field across the nanopore;
(e) translocating at a rate of less than 1000 nucleotides per second (nt/sec) the labeled extension strand of the captured double stranded product through the nanopore by the applied electrical field, wherein the translocating strand of the double stranded product is unzipped as it enters the nanopore;
(f) detecting optical signals from the optical labels as said labeled extension strand passes through said nanopore; and
(g) determining a nucleotide sequence of the nucleic acid from the optical signals detected in step (f).

2. The method of claim 1 further including the step of isolating said double stranded product prior to said step of disposing said double stranded product in said first chamber.

3. The method of claim 1, wherein said at least one nanopore comprises an array of a plurality of nanopores.

4. The method of claim 3, wherein said plurality is at least 100.

5. The method of claim 1 further including steps of disposing in said first chamber a predetermined standard comprising a known double stranded portion and a 5' non-complementary tail as a single stranded overhang; and selecting an electric field strength to apply across said at least one nanopore that corresponds to said rate of translocation of less than 1000 nucleotides per second.

6. The method of claim 1, wherein said double stranded product further comprises a 3' overhang at the same end of said double stranded product as said 5' non-complementary tail and wherein the 3' overhang is shorter in length than said 5' non-complementary tail.

7. The method of claim 1, wherein said double stranded product further comprises a 3' overhang at the same end of said double stranded product as said 5' non-complementary tail and wherein said method further includes a step of treating said double stranded product with a 3' single stranded exonuclease to remove the 3' overhang prior to said step of capturing.

8. A method of analyzing a nucleic acid comprising:
(a) extending a primer on a template in a reaction mixture to produce a double stranded product comprising a labeled extended strand with a free 3'-hydroxyl, wherein said labels of said labeled extended strand are optical labels each capable of generating an optical signal indicative of a nucleotide to which it is attached;
(b) extending further the extended strand without a template with a terminal transferase activity to produce a 3'-single stranded tail on the double stranded product;
(c) providing at least one nanopore that separates and provides fluid communication between a first chamber and a second chamber, wherein each nanopore of the at least one nanopore is capable of passing a single stranded nucleic acid but not a double stranded nucleic acid;
(d) disposing double stranded product with the 3'single stranded tails in the first chamber;
(e) capturing a 3' single stranded tail of a double stranded product by the at least one nanopore by applying an electrical field across the nanopore;
(f) translocating at a rate of less than 1000 nucleotides per second (nt/sec) the labeled extension strand of the captured double stranded product through the nanopore by the applied electrical field, wherein the translocating strand of the double stranded product is unzipped as it enters the nanopore;
(g) detecting optical signals from the optical labels as said labeled extension strand passes through said nanopore; and
(h) determining a nucleotide sequence of the nucleic acid from the optical signals detected in step (g).

9. A method of determining a nucleotide sequence of a polynucleotide, the method comprising the steps of:
(a) providing a labeled double stranded products of target polynucleotides, wherein a labeled strand of each labeled double stranded product comprises a single stranded overhang and wherein different kinds of nucleotides of the labeled strand have different optical labels that generate distinct optical signals;
(b) providing at least one nanopore that provides fluid communication between a first chamber and a second chamber, wherein each nanopore is capable of passing a single stranded nucleic acid but not a double stranded nucleic acid;
(c) disposing the labeled double stranded product in the first chamber;
(d) capturing by the at least one nanopore a single stranded overhang of a labeled double stranded product by applying an electrical field across the nanopore;
(e) translocating the labeled stand through the nanopore so that the nucleotides of the labeled strand pass single file through an excitation zone at a rate of less than 1000 nucleotides per second (nt/sec), wherein optical labels are excited to generate optical signals and wherein the translocating labeled strand of the double stranded product is unzipped as it enters the nanopore;
(f) detecting a time series of optical signals from the optical labels as the labeled strand translocates through the nanopore to produce a strand optical signature; and
(g) determining a sequence of the target polynucleotide from the strand optical signature.

10. The method of claim 9, wherein said single stranded overhang is a 3'-overhang.

11. The method of claim 9, wherein said single stranded overhang is a 5'-overhang.

## Patentansprüche

1. Verfahren zum Analysieren einer Nukleinsäure mit den Schritten:
(a) Verlängern eines Primers mit einem nicht-komplementären 5'-Schwanz auf einer Matrize in einem Reaktionsgemisch, um ein doppelsträngiges Produkt zu erzeugen, das einen markierten verlängerten Strang und den nicht-komplementären 5'-Schwanz als einzelsträngigen Überhang umfasst, wobei die Markierungen des markierten verlängerten Strangs optische Markierungen sind, die jeweils in der Lage sind, ein optisches Signal zu erzeugen, das auf ein Nukleotid hinweist, an das sie gebunden sind;
(b) Bereitstellen mindestens einer Nanopore, die eine Flüssigkeitsverbindung zwischen einer ersten Kammer und einer zweiten Kammer herstellt, wobei jede Nanopore der mindestens einen Nanopore in der Lage ist, eine einzelsträngige Nukleinsäure, nicht aber eine doppelsträngige Nukleinsäure durchzulassen;
(c) Einbringen des doppelsträngigen Produkts in die erste Kammer;
(d) Einfangen des 5'-nicht-komplementären Schwanzes des doppelsträngigen Produkts durch die mindestens eine Nanopore durch Anlegen eines elektrischen Feldes an die Nanopore;
(e) Translozieren des markierten verlängerten Strangs des eingefangenen Doppelstrangprodukts durch das angelegte elektrische Feld mit einer Rate von weniger als 1000 Nukleotiden pro Sekunde (nt/sec) durch die Nanopore, wobei der translozierende Strang des doppelsträngigen Produkts beim Eintritt in die Nanopore abgetrennt wird;
(f) Erfassen optischer Signale von den optischen Markierungen, wenn der markierte verlängerte Strang durch die Nanopore hindurchgeht; und
(g) Bestimmen einer Nukleotidsequenz der Nukleinsäure aus den in Schritt (f) erfassten optischen Signalen.

2. Verfahren nach Anspruch 1, das ferner den Schritt des Isolierens des doppelsträngigen Produkts vor dem Schritt des Einbringens des doppelsträngigen Produkts in die erste Kammer umfasst.

3. Verfahren nach Anspruch 1, bei dem die mindestens eine Nanopore eine Anordnung aus einer Mehrzahl von Nanoporen umfasst.

4. Verfahren nach Anspruch 3, bei dem die Mehrzahl mindestens 100 beträgt.

5. Verfahren nach Anspruch 1, das ferner die Schritte des Einbringens eines vorgegebenen Standards in die erste Kammer, der einen bekannten doppelsträngigen Abschnitt und einen nicht-komplementären 5'-Schwanz als einzelsträngigen Überhang umfasst; und des Auswählens einer elektrischen Feldstärke, die über die mindestens eine Nanopore anzulegen ist und die der Translokationsrate von weniger als 1000 Nukleotiden pro Sekunde entspricht, umfasst.

6. Verfahren nach Anspruch 1, bei dem das doppelsträngige Produkt ferner einen 3'-Überhang am gleichen Ende des doppelsträngigen Produkts wie der nicht-komplementäre 5'-Schwanz aufweist und bei dem der 3'-Überhang kürzer ist als der nicht-komplementäre 5'-Schwanz.

7. Verfahren nach Anspruch 1, bei dem das doppelsträngige Produkt ferner einen 3'-Überhang am gleichen Ende des doppelsträngigen Produkts wie der nicht-komplementäre 5'-Schwanz aufweist und bei dem das Verfahren ferner einen Schritt des Behandelns des doppelsträngigen Produkts mit einer einzelsträngigen 3'-Exonuklease umfasst, um den 3'-Überhang vor dem Schritt des Einfangens zu entfernen.

8. Verfahren zum Analysieren einer Nukleinsäure mit den Schritten:
(a) Verlängern eines Primers auf einer Matrize in einem Reaktionsgemisch, um ein doppelsträngiges Produkt zu erzeugen, das einen markierten verlängerten Strang mit einem freien 3'-Hydroxyl umfasst, wobei die Markierungen des markierten verlängerten Strangs optische Markierungen sind, die jeweils in der Lage sind, ein optisches Signal zu erzeugen, das auf ein Nukleotid hinweist, an das sie gebunden sind;
(b) weiteres Verlängern des verlängerten Strangs ohne eine Matrize mit einer Terminal-Transferaseaktivität, um an dem doppelsträngigen Produkt einen einzelsträngigen 3'-Schwanz zu erzeugen;
(c) Bereitstellen mindestens einer Nanopore, die eine erste Kammer und eine zweite Kammer trennt und eine Flüssigkeitsverbindung zwischen ihnen herstellt, wobei jede Nanopore der mindestens einen Nanopore in der Lage ist, eine einzelsträngige Nukleinsäure, nicht aber eine doppelsträngige Nukleinsäure durchzulassen;
(d) Einbringen des doppelsträngigen Produkts mit den einzelsträngigen 3'-Schwänzen in die erste Kammer;
(e) Einfangen eines einzelsträngigen 3'-Schwanzes eines doppelsträngigen Produkts durch die mindestens eine Nanopore durch Anlegen eines elektrischen Feldes an die Nanopore;
(f) Translozieren des markierten verlängerten Strangs des eingefangenen doppelsträngigen Produkts durch das angelegte elektrische Feld mit einer Rate von weniger als 1000 Nukleotiden pro Sekunde (nt/sec) durch die Nanopore, wobei der translozierende Strang des doppelsträngigen Produkts beim Eintritt in die Nanopore abgetrennt wird;
(g) Erfassen optischer Signale von den optischen Markierungen, wenn der markierte verlängerte Strang durch die Nanopore hindurchgeht; und
(h) Bestimmen einer Nukleotidsequenz der Nukleinsäure aus den in Schritt (g) erfassten optischen Signalen.

9. Verfahren zur Bestimmung einer Nukleotidsequenz eines Polynukleotids, wobei das Verfahren die folgenden Schritte umfasst:
(a) Bereitstellen eines markierten doppelsträngigen Produkts von Zielpolynukleotiden, wobei ein markierter Strang jedes markierten doppelsträngigen Produkts einen einzelsträngigen Überhang aufweist und wobei verschiedene Arten von Nukleotiden des markierten Strangs verschiedene optische Markierungen aufweisen, die unterschiedliche optische Signale erzeugen;
(b) Bereitstellen von mindestens einer Nanopore, die eine Flüssigkeitsverbindung zwischen einer ersten Kammer und einer zweiten Kammer herstellt, wobei jede Nanopore in der Lage ist, eine einzelsträngige Nukleinsäure, nicht aber eine doppelsträngige Nukleinsäure durchzulassen;
(c) Einbringen des markierten doppelsträngigen Produkts in die erste Kammer;
(d) Einfangen eines einzelsträngigen Überhangs eines markierten doppelsträngigen Produkts durch die mindestens eine Nanopore durch Anlegen eines elektrischen Feldes an die Nanopore;
(e) Translozieren des markierten Strangs durch die Nanopore, so dass die Nukleotide des markierten Strangs eine Anregungszone einzeln mit einer Rate von weniger als 1000 Nukleotiden pro Sekunde (nt/sec) durchlaufen, wobei optische Markierungen angeregt werden, um optische Signale zu erzeugen, und wobei der translozierende markierte Strang des doppelsträngigen Produkts bei seinem Eintritt in die Nanopore abgetrennt wird;
(f) Erfassen einer Zeitreihe optischer Signale von den optischen Markierungen, wenn der markierte Strang durch die Nanopore transloziert, um eine optische Signatur des Strangs zu erzeugen; und
(g) Bestimmen einer Sequenz des Ziel-Polynukleotids aus der optischen Signatur des Strangs.

10. Verfahren nach Anspruch 9, bei dem der einzelsträngige Überhang ein 3'-Überhang ist.

11. Verfahren nach Anspruch 9, bei dem der einzelsträngige Überhang ein 5'-Überhang ist.

## Revendications

1. Procédé d'analyse d'un acide nucléique comprenant les étapes consistant à :
(a) étendre une amorce ayant une queue non complémentaire en 5' sur une matrice dans un mélange réactionnel pour produire un produit double brin comprenant un brin étendu marqué et la queue non complémentaire en 5' en tant que surplomb simple brin, où les marqueurs dudit brin étendu marqué sont des marqueurs optiques qui sont chacun capables de générer un signal optique indiquant un nucléotide auquel il est attaché ;
(b) fournir au moins un nanopore qui assure une communication fluidique entre une première chambre et une deuxième chambre, où chaque nanopore de l'au moins un nanopore est capable de faire passer un acide nucléique simple brin mais pas un acide nucléique double brin ;
(c) disposer le produit double brin dans la première chambre ;
(d) capturer, par l'au moins un nanopore, la queue non complémentaire en 5' du produit double brin en appliquant un champ électrique à travers le nanopore ;
(e) transloquer, à une vitesse inférieure à 1000 nucléotides par seconde (nt/sec), le brin d'extension marqué du produit double brin capturé à travers le nanopore par le champ électrique appliqué, où le brin de translocation du produit double brin est dégrafé à mesure qu'il entre dans le nanopore ;
(f) détecter des signaux optiques à partir des marqueurs optiques à mesure que ledit brin d'extension marqué passe à travers ledit nanopore ; et
(g) déterminer une séquence nucléotidique de l'acide nucléique à partir des signaux optiques détectés dans l'étape (f).

2. Procédé de la revendication 1, comportant en outre l'étape consistant à isoler ledit produit double brin avant ladite étape consistant à disposer ledit produit double brin dans ladite première chambre.

3. Procédé de la revendication 1, dans lequel ledit au moins un nanopore comprend un réseau d'une pluralité de nanopores.

4. Procédé de la revendication 3, dans lequel ladite pluralité est d'au moins 100.

5. Procédé de la revendication 1, comportant en outre les étapes consistant à disposer, dans ladite première chambre, un témoin prédéterminé comprenant une partie double brin connue et une queue non complémentaire en 5' en tant que surplomb simple brin ; et à sélectionner une intensité de champ électrique à appliquer à travers ledit au moins un nanopore qui correspond à ladite vitesse de translocation qui est inférieure à 1000 nucléotides par seconde.

6. Procédé de la revendication 1, dans lequel ledit produit double brin comprend en outre un surplomb en 3' au niveau de la même extrémité dudit produit double brin que ladite queue non complémentaire en 5' et dans lequel le surplomb en 3' a une longueur plus courte que celle de ladite queue non complémentaire en 5'.

7. Procédé de la revendication 1, dans lequel ledit produit double brin comprend en outre un surplomb en 3' au niveau de la même extrémité dudit produit double brin que ladite queue non complémentaire en 5' et ledit procédé comportant en outre une étape consistant à traiter ledit produit double brin avec une exonucléase simple brin en 3' pour éliminer le surplomb en 3' avant ladite étape de capture.

8. Procédé d'analyse d'un acide nucléique comprenant les étapes consistant à :
(a) étendre une amorce sur une matrice dans un mélange réactionnel pour produire un produit double brin comprenant un brin étendu marqué avec un 3'-hydroxyle libre, où lesdits marqueurs dudit brin étendu marqué sont des marqueurs optiques qui sont chacun capables de générer un signal optique indiquant un nucléotide auquel il est attaché ;
(b) étendre davantage le brin étendu sans matrice avec une activité transférase terminale pour produire une queue simple brin en 3' sur le produit double brin ;
(c) fournir au moins un nanopore qui sépare et assure une communication fluidique entre une première chambre et une deuxième chambre, où chaque nanopore de l'au moins un nanopore est capable de faire passer un acide nucléique simple brin mais pas un acide nucléique double brin ;
(d) disposer le produit double brin avec les queues simple brin en 3' dans la première chambre ;
(e) capturer une queue simple brin en 3' d'un produit double brin par l'au moins un nanopore en appliquant un champ électrique à travers le nanopore ;
(f) transloquer, à une vitesse inférieure à 1000 nucléotides par seconde (nt/sec), le brin d'extension marqué du produit double brin capturé à travers le nanopore par le champ électrique appliqué, où le brin de translocation du produit double brin est dégrafé à mesure qu'il entre dans le nanopore ;
(g) détecter des signaux optiques à partir des marqueurs optiques à mesure que ledit brin d'extension marqué passe à travers ledit nanopore ; et
(h) déterminer une séquence nucléotidique de l'acide nucléique à partir des signaux optiques détectés dans l'étape (g).

9. Procédé de détermination d'une séquence nucléotidique d'un polynucléotide, le procédé comprenant les étapes consistant à :
(a) fournir des produits double brin marqués de polynucléotides cibles, où un brin marqué de chaque produit double brin marqué comprend un surplomb simple brin et où différents types de nucléotides du brin marqué ont des marqueurs optiques différents qui génèrent des signaux optiques distincts ;
(b) fournir au moins un nanopore qui assure une communication fluidique entre une première chambre et une deuxième chambre, où chaque nanopore est capable de faire passer un acide nucléique simple brin mais pas un acide nucléique double brin ;
(c) disposer le produit double brin marqué dans la première chambre ;
(d) capturer, par l'au moins un nanopore, un surplomb simple brin d'un produit double brin marqué en appliquant un champ électrique à travers le nanopore ;
(e) transloquer le brin marqué à travers le nanopore de sorte que les nucléotides du brin marqué passent en file indienne à travers une zone d'excitation à une vitesse inférieure à 1000 nucléotides par seconde (nt/sec), où les marqueurs optiques sont excités pour générer des signaux optiques et où le brin marqué de translocation du produit double brin est dégrafé à mesure qu'il entre dans le nanopore ;
(f) détecter une série temporelle de signaux optiques à partir des marqueurs optiques à mesure que le brin marqué transloque à travers le nanopore pour produire une signature optique de brin ; et
(g) déterminer une séquence du polynucléotide cible à partir de la signature optique de brin.

10. Procédé de la revendication 9, dans lequel ledit surplomb simple brin est un surplomb en 3'.

11. Procédé de la revendication 9, dans lequel ledit surplomb simple brin est un surplomb en 5'.
